(19) Europäisches Patentamt
European Patent Office
Office européen des brevets

(11) Publication number: 0 126 005
B1

(12) # EUROPEAN PATENT SPECIFICATION

(45) Date of publication of patent specification: 23.09.87

(21) Application number: 84400973.8

(22) Date of filing: 11.05.84

(51) Int. Cl.[4]: C 07 D 493/08 // (C07D493/08, 307:00)

(54) 7-Oxabicycloheptane prostaglandin intermediates and method for preparing same.

(30) Priority: 13.05.83 US 494232
13.05.83 US 494233
13.05.83 US 494235

(43) Date of publication of application:
21.11.84 Bulletin 84/47

(45) Publication of the grant of the patent:
23.09.87 Bulletin 87/39

(84) Designated Contracting States:
AT BE CH DE FR GB IT LI LU NL SE

(56) References cited:
FR-A-2 497 203
GB-A-2 062 629

CHEMICAL ABSTRACTS, vol. 90, no. 9, February 26, 1979, Columbus, Ohio, USA EGGELTE, TEUNIS A.; DE KONING, HENK; HUISMAN, HENDERIKUS O. "Synthesis of 9,11-epoxy-9a-homoprostaglandin analogs." page 479, column 2, abstract no. 71 794f

(73) Proprietor: E.R. Squibb & Sons, Inc.
Lawrenceville-Princeton Road
Princeton, N.J. 08540 (US)

(72) Inventor: Sprague, Peter W.
23 Timberlane Drive
Pennington New Jersey (US)
Inventor: Heikes, James E.
2 Kent Lane
East Windsor New Jersey (US)

(74) Representative: Maiffret, Bernard et al
Law Offices of William J. Rezac
49 avenue Franklin D. Roosevelt
F-75008 Paris (FR)

(56) References cited:
CHEMICAL ABSTRACTS, vol. 92, no. 11, March 17, 1980, Columbus, Ohio, USA GRANDGUILLOT, JEAN CLAUDE; ROUESSAC, FRANCIS. "Study of a synthetic route to 5-alkyl-2(5H)-furanones." page 569, column 1, abstract no. 94 159h

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 126 005 B1

(56) References cited:
CHEMICAL ABSTRACTS, vol. 90, no. 15, April 9, 1979, Columbus, Ohio, USA SUZUKI, TOSHIO; TOMINO, AKIKO; KAGAYA, SEIKO; UNNO, KATSUO; KAMETANI, TETSUJI; IHARA, MASATAKA; TAKAHASHI, TAMIKO "Correction on the structure of Diels-Alder reaction products of furan derivatives with maleic anhydride." page 633, column 2, abstract no. 121 460s

**Description**

The present invention relates to optically active intermediates for use in preparing 7-oxabicycloheptane prostaglandin analogs and to a method for preparing such intermediates.

US—A—4,413,054 to Sprague dated March 6, 1979 discloses 7-oxabicycloheptane and 7-oxabicycloheptene prostaglandin analogs which are prepared by the following methods.

In a first method maleic anhydride is made to react with an unsubstituted or substituted furan of the formula

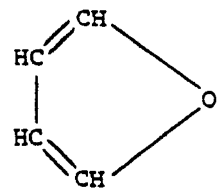

to form a compound of the formula

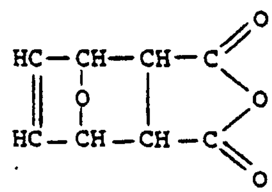

which is reduced to form

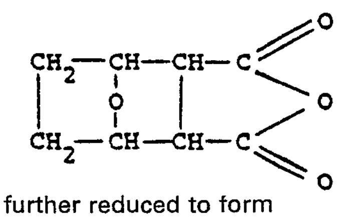

The above compound is then further reduced to form

*A*. 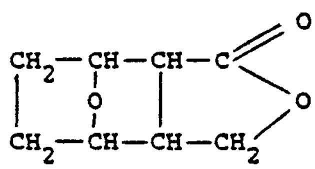

Treatment of the above compound with diisobutylaluminum hydride or diisobutylborane yields

*B* 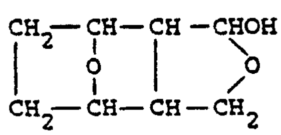

Submitting compound *A* to Wittig reaction conditions produces

*C* 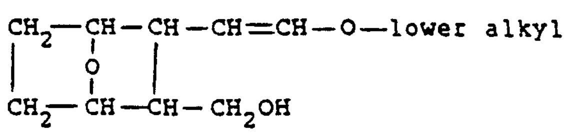

Compound *C* is then acylated and then hydrolyzed to form the aldehyde

*D* 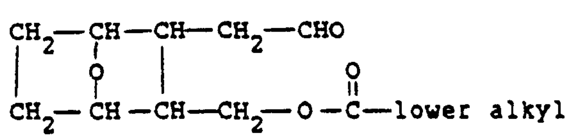

All of the above compounds are in the form of racemic mixtures.

Aldehyde *D* is subjected to a Wittig reaction to form a compound of the structure

*E* 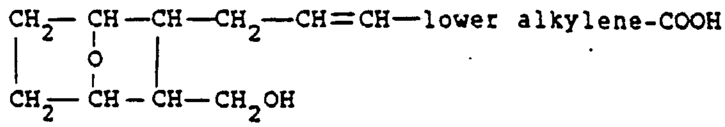

which is esterified to form the corresponding lower alkyl ester. The hydroxymethyl group in the 3-position of the ester is then oxidized to obtain the aldehyde

*F* 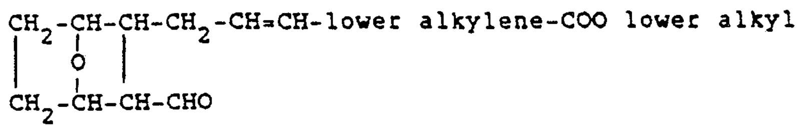

Aldehyde *F* which is in the form of a racemic mixture is employed to form 7-oxabicycloheptane prostaglandin analogs.

In accordance with the present invention, a method is provided for forming the aldehyde *F* also depicted graphically as

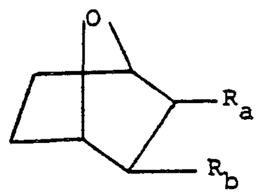

wherein one of $R_a$ and $R_b$ is $—CH_2—CH=CH—$ lower alkylene —COO lower alkyl and the other is —CHO in the form of its optically active isomer as opposed to a racemic mixture of two enantiomers as disclosed by Sprague in US—A—4,143,054. The optically active aldehyde F(a) is then employed to form optically active 7-oxabicycloheptane prostaglandin analogs, for example, using the technique described by Sprague in US—A—4,143,054.

In carrying out the method of the invention as described hereinafter several novel optically active intermediates are formed having the following formula

I 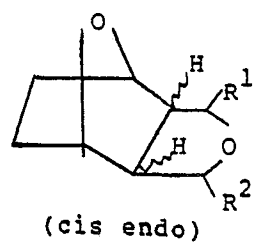

(cis endo)

wherein one of $R^1$ and $R^2$ is

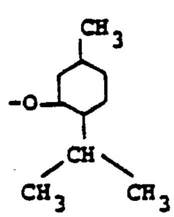 or 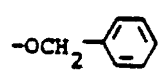

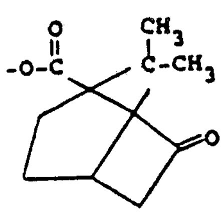 or 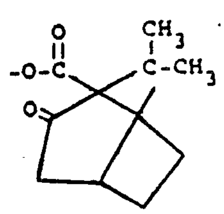

and the other is hydrogen, and includes the following compounds:

II

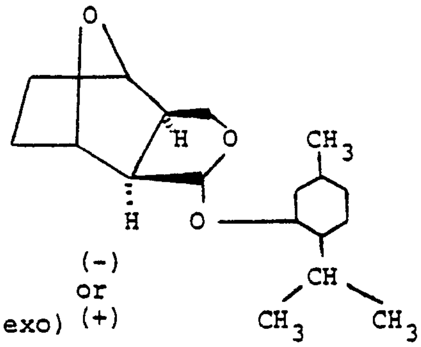

(-) or (+)

(cis exo)

[3aR-[1-(1R,2S,5R),3aα,4α,7α,7aα]]-Octahydro-1-[[5-methyl-2-(1-methylethyl)cyclohexyl]oxy]-4,7-epoxyisobenzofuran

III

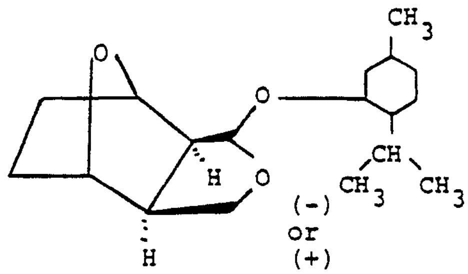

(-) or (+)

(cis exo)

[3aS-[1-(1R,2S,5R),3aα,4α,7α,7aα]]-Octahydro-1-[[5-methyl-2-(1-methylethyl)cyclohexyl]oxy]-4,7-epoxyisobenzofuran

IV

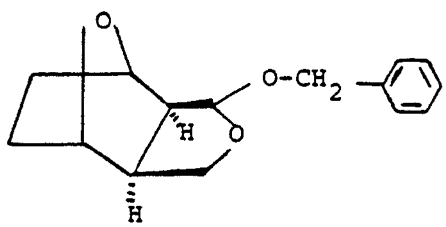

or

(cis exo)

[3aS-(3aα,4α,7α)]-Octahydro-1-benzyloxy-4,7-epoxy-isobenzofuran

IVa

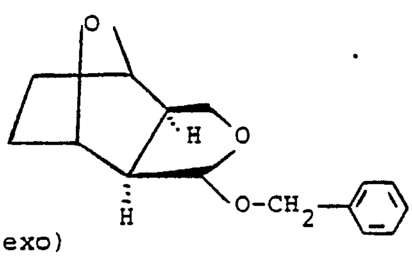

(cis-exo)

[3aR-(3aα,4α,7α,7aα)]-Octahydro-1-benzyloxy-4,7-epoxy-isobenzofuran

V

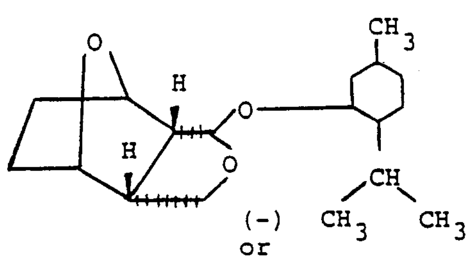

(-) or (+)

(cis endo)

[3aR-[1-(1R,2S,5R),3aα,4β,7β,7aα]]-Octahydro-1-[[5-methyl-2-(1-methylethyl)cyclohexyl]oxy]-4,7-epoxyisobenzofuran

VI

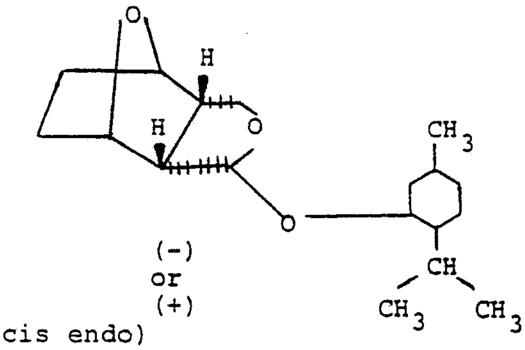

(-)
or
(+)

(cis endo)

[3aS-[1-(1R,2S,5R),3aα,4β,7aα]]-Octahydro-1-[[5-methyl-2-(1-methylethyl)cyclohexyl]oxy]-4,7-epoxyisobenzofuran

VII

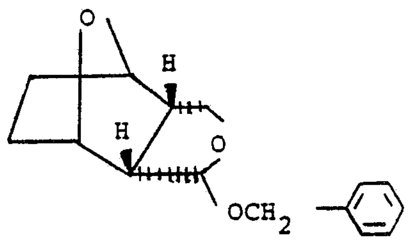

or

(cis endo)

[3aS-(3aα,4β,7aα)]-Octahydro-1-benzyloxy-4,7-epoxyisobenzofuran

VIIa

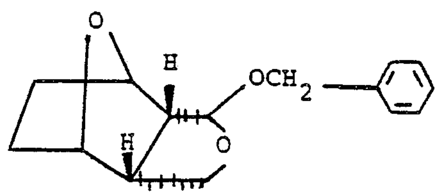

(cis endo)

[3aR-(3aα,4β,7β,7aα]-Octahydro-1-benzyloxy-4,7-epoxyisobenzofuran

VIII

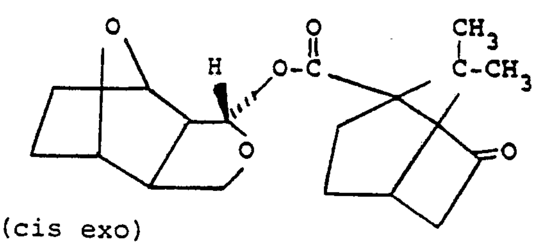

(cis exo)

[1S-[1α(1S),3aα,4α,7α,7aα]]-Octahydro-1-[[2-oxo-7,7-dimethylbicyclo[2.2.1]heptane]carboxyl]-4,7-epoxyisobenzofuran

IX

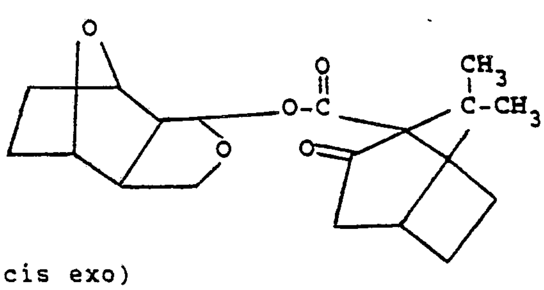

(cis exo)

[1S-[1α(1R), 3aα,4α,7α,7aα]]-Octahydro-1-[[2-oxo-7,7-dimethylbicyclo[2.2.1]heptane]carboxyl]-4,7-epoxyisobenzofuran

X

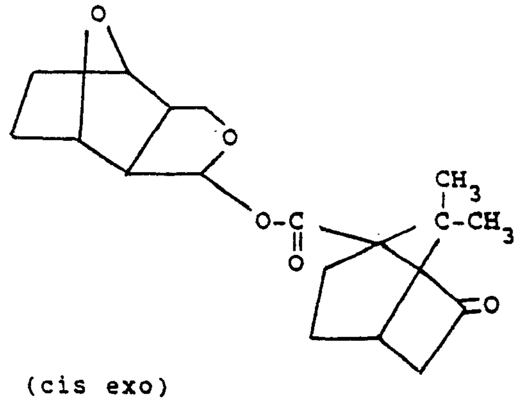

(cis exo)

[1R-[1α(1S), 3aα,4α,7α,7aα]]-Octahydro-1-[[2-oxo-7,7-dimethylbicyclo[2.2.1]heptane]carboxyl]-4,7-epoxyisobenzofuran

XI

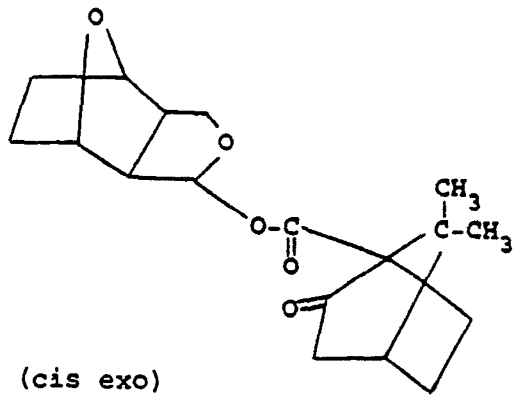

(cis exo)

[1R-[1α(1R),3aα,4α,7α,7aα]]-Octahydro-1-[[2-oxo-7,7-dimethylbicyclo[2.2.1]heptane]carboxyl]-4,7-epoxyisobenzofuran

Also included among the novel optically active intermediates formed during the method of this invention are compounds having the following formula:

XII

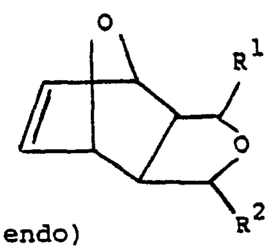

(cis endo)

(depicted graphically as

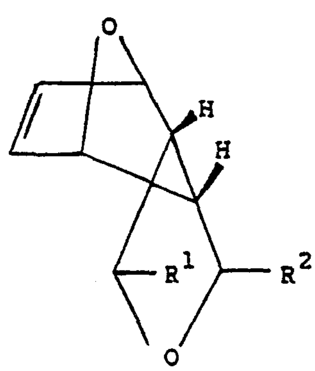

)

wherein one of $R^1$ and $R^2$ is

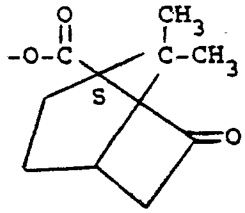

or

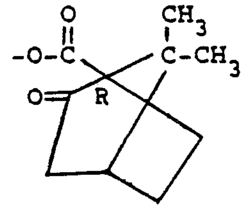

and the other is H, and includes the following compounds:

XIII

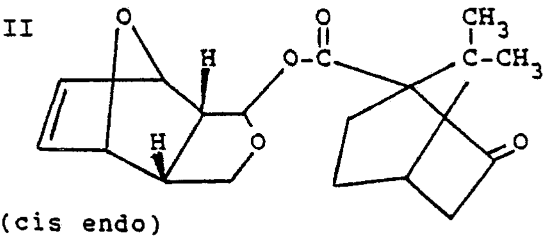

(cis endo)

[1S-[1α(1S),3aα,4β,7β,7aα]]-1,3,3a,4,7,7a-Hexahydro-1-[[2-oxo-7,7-dimethylbicyclo[2.2.1]-heptane]carboxyl]-4,7-epoxyisobenzofuran

XIV

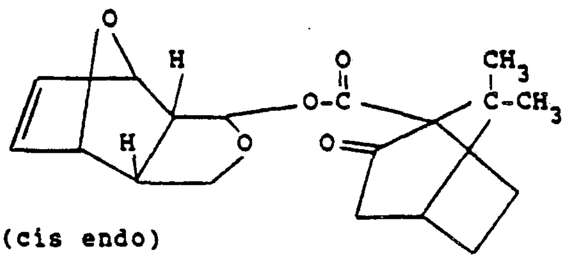

(cis endo)

[1S-[1α(1R),3aα,4β,7β,7aα]]-1,3,3a,4,7,7a-Hexahydro-1-[[2-oxo-7,7-dimethylbicyclo[2.2.1]-heptane]carboxyl]-4,7-epoxyisobenzofuran

XV

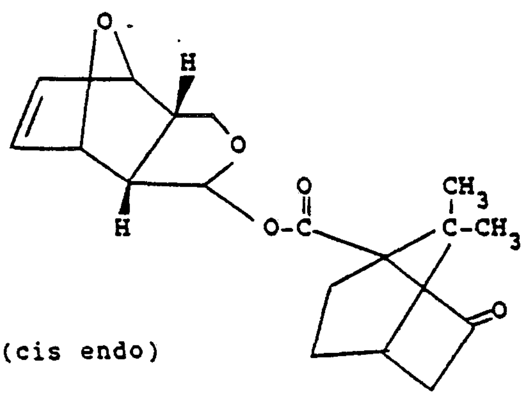

(cis endo)

[1R-[1α(1S),3aα,4β,7β,7aα]]-1,3,3a,4,7,7a-Hexahydro-1-[[2-oxo-7,7-dimethylbicyclo[2.2.1]-heptane]carboxyl]-4,7-epoxyisobenzofuran

XV I

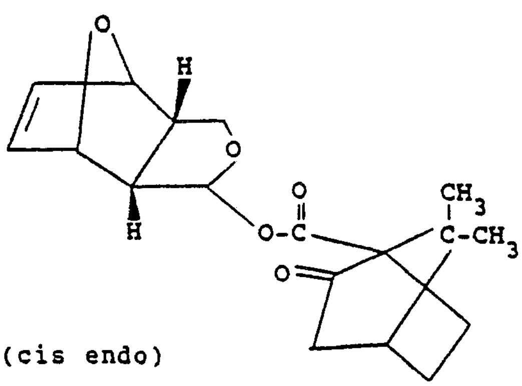

(cis endo)

[1R-[1α(1R),3aα,4β,7β,7aα]]-1,3,3a,4,7,7a-Hexahydro-1-[[2-oxo-7,7-dimethylbicyclo[2.2.1]-heptane]carboxyl]-4,7-epoxyisobenzofuran

The method of the present invention for forming optically active or chiral intermediates for use in preparing optically active 7-oxabicycloheptane prostaglandin analogs may be summarized in the following reaction sequences.

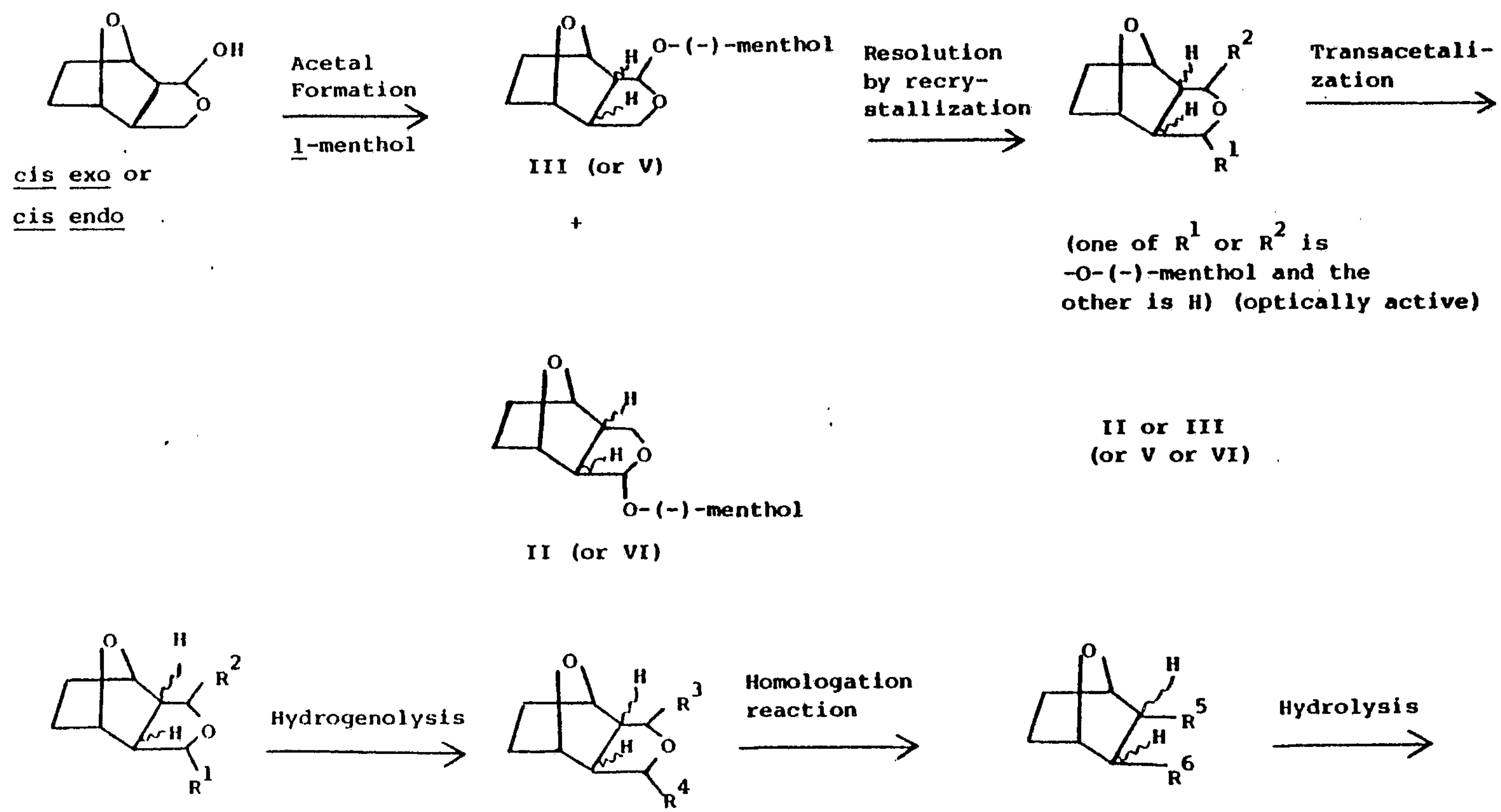


cis exo or
cis endo

III (or V)

+

II (or VI)

(one of $R^1$ or $R^2$ is -O-(-)-menthol and the other is H) (optically active)

II or III
(or V or VI)

(one of $R^1$ or $R^2$ is $-O-CH_2-$phenyl and the other is H)
(optically active)
IV or VII

(one of $R^3$ and $R^4$ is OH and the other is H)
(optically active)
J

(one of $R^5$ or $R^6$ is $CH_2OH$ and the other is $-CH=CH-OCH_3$)
(optically active)
K

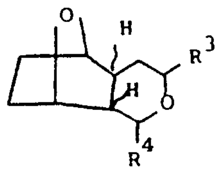

(wherein one of $R^3$ and $R^4$ is OH and the other is H)
(optically active)

L

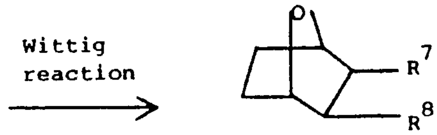


(one of $R^7$ and $R^8$ is $-CH_2-CH=CH-$lower alkylene$-COCH_3$ and the other is $-CH_2OH$)
(optically active)

M

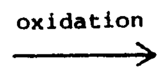


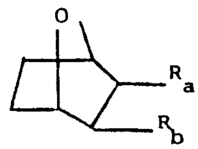

(one of $R_a$ and $R_b$ is $-CH_2-CH=CH-$lower alkylene$-CO_2CH_3$ and the other is $-CHO$)
(optically active)

N

The reaction sequence for preparing the optically active antipodes or mirror images of compounds of formulae II, III and IV, namely compounds of formulae V, VI and VII, respectively, may be prepared following the above reaction sequence except that *d*-menthol is employed in place of the corresponding *l*-isomer.

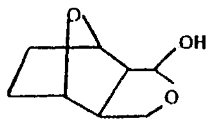

G

cis exo or
cis endo

Acetal
Formation

1-menthol

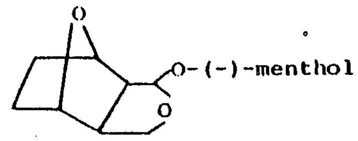

H

(100% optically pure)

+

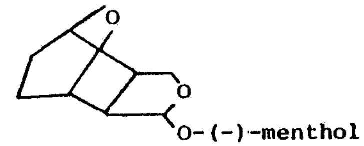

J

(90% optically pure)

Resolution
by recry-
stallization

J

Transacetali-
zation

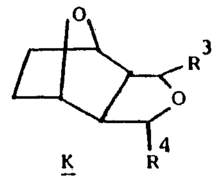

K

($R^4$ is -O-CH$_2$- 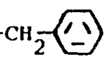
and $R^3$ is H)
(optically active)

Hydrogenolysis

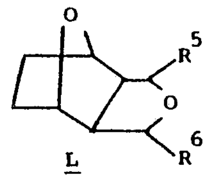

L

($R^6$ is OH and $R^5$
is H)
(90% optically pure)

Ketopinate
ester
formation

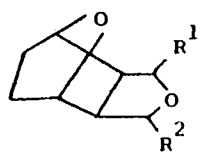

(wherein $R^2$ is

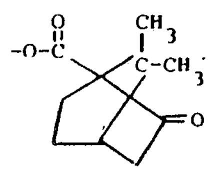

or

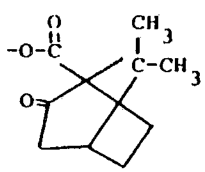

and $R^1$ is H)

(optically active)

Hydrolysis

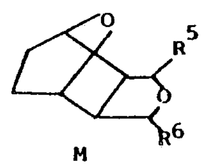

M

(100% optically
active)
($R^6$ is OH and $R^5$
is H)

Homologation
reaction

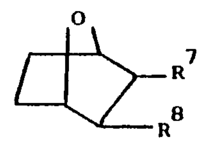

N

($R^7$ is
$CH_2OH$ and
$R^8$ is $-CH=CH-OCH_3$)
(optically active)

Hydrolysis

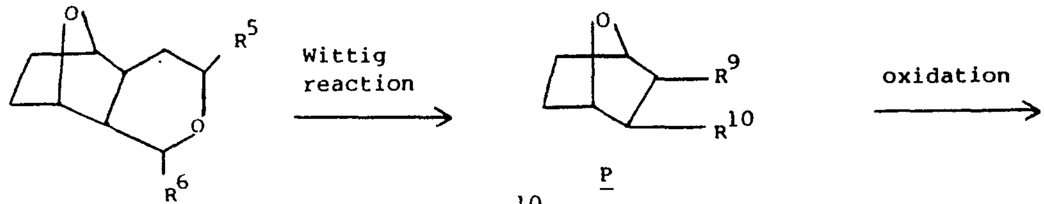


O

(wherein $R^6$ is OH and $R^5$ is H) (optically active)

P

($R^{10}$ is $-CH_2-CH=CH-$lower alkylene$-COCH_3$ and $R^9$ is $-CH_2OH$) (optically active)

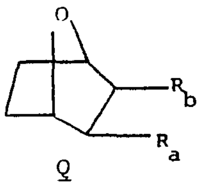

Q

(one of $R_a$ is $-CH_2-CH=CH-$lower alkylene$-CO_2CH_3$ and $R_b$ is $-CHO$) (optically active)

The method of the present invention for forming optically active or chiral intermediates for use in preparing optically active 7-oxabicycloheptane prostaglandin analogs includes the step of reacting the racemic hemiacetal

*G* 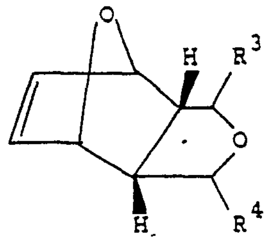

wherein one of $R^3$ and $R^4$ is OH and the other is H (prepared as described in US—A—4,143,054) with *l*-ketopinic acid halide, such as the chloride (prepared as described in U.S. Patent No. 4,235,891) employing a molar ratio of *G*:ketopinic acid chloride of about 0.5:1 to about 1:1, in the presence of 4-dimethylaminopyridine or 4-diethylaminopyridine and an inert solvent such as pyridine or dichloromethane to form a diastereomeric mixture of the ketopinate esters II and IV (where *l*-ketopinic acid halide is used). The mixture of diastereomers of formula I is recrystallized, for example, from isopropyl ether to form the optically active isomer II. The reaction sequence for preparing the optically active antipodes or mirror images of compounds of formulae II and IV, namely compounds of formulae III and V, respectively, may be prepared following the above reaction sequence except that *d*-ketopinic acid halide is employed in place of the corresponding *l*-isomer.

The optically active ketopinate II (IV) or III(V) may then be subjected to ester hydrolysis, for example, by reacting the ketopinate with lithium hydroxide/hydrogen peroxide, to form the optically active hemiacetal

*H* 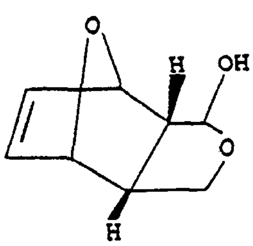 (or *H′* 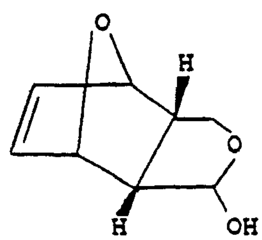 )

which then undergoes dimethylhydrazone formation by reaction with unsymdimethylhydrazine to form the dimethylhydrazone

*J* 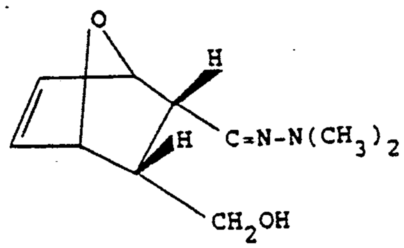 (or *J'* 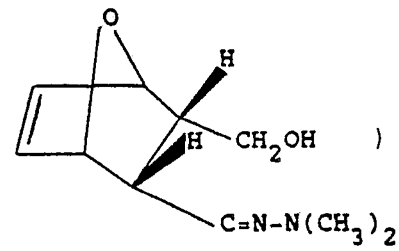 )

Compound *J* is then acetylated to form

*K* 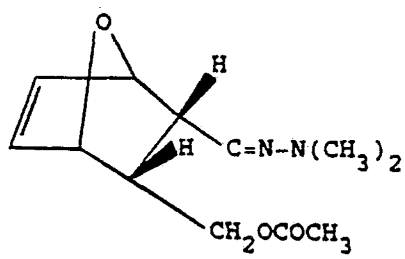 (or K' 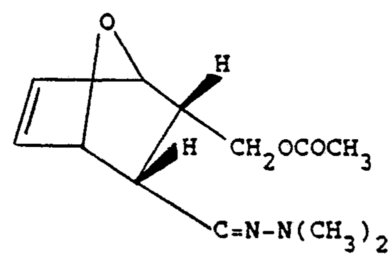 )

which is reduced by reaction with hydrogen in the presence of a catalyst such as palladium on carbon to form

*L* 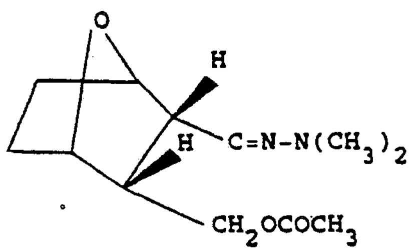 (or L' 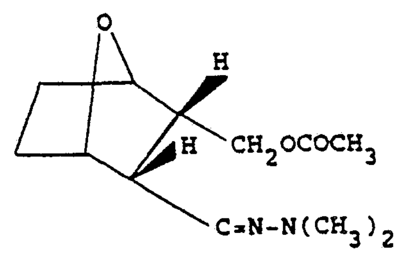 )

Compound *L* is then made to undergo hydrazone hydrolysis, for example, by reacting *L* with cupric chloride dihydrate in the presence of a solvent such as tetrahydrofuran to form the aldehyde

*M* 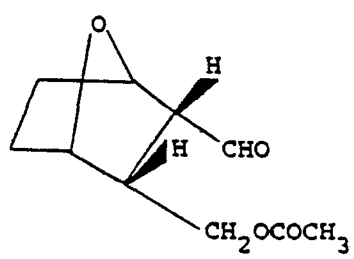 (or M' 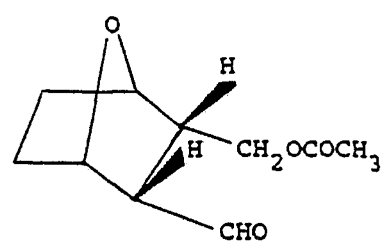 )

The prostaglandin aldehyde analog *M* (and *M'* where *d*-ketopinic acid halide is used) may then be employed to prepare 7-oxabicycloheptane prostaglandins following the procedure as set out in US—A—4,143,054 to Sprague. Such prostaglandin derivatives are useful in the treatment of thrombolytic disease as explained in the above Sprague patent.

The method of the invention is an advance over prior art methods for forming optically active 7-oxabicycloheptane prostaglandin analogs in that the resolution is performed at a very early stage of the synthesis, preferably on a hemi-acetal intermediate. In the present method, the undesired menthol diastereomer can be separated out and recycled. Thus, in principle, the hemi-acetal intermediate is transformed to a single diastereomer.

In carrying out the method of the invention, referring to the first reaction sequence described herein, the racemic *cis-exo* or *cis-endo* hemiacetal

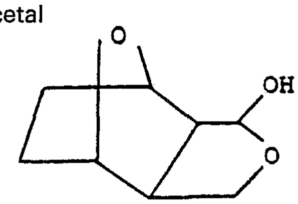

is converted to a diastereomeric mixture of menthol compounds III (or V) and II (or VI) by reacting same with *l*-menthol in the presence of trace amounts of p-toluenesulfonic acid and an inert solvent such as benzene at reflux temperatures under an inert atmosphere, for example, nitrogen to form a mixture of diastereomers of formula II or III (or V or VI) which is recrystallized, for example, from methylene chloride-ethyl acetate and thus resolved to form the optically active acetal isomer II or III

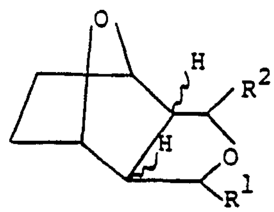

(where one of $R^1$ and $R^2$ is -O-menthol and the other is H).

The acetal II or III (or V or VI) is allowed to react with benzyl alcohol at elevated temperatures of at least 110° to form the optically active benzyloxy compound

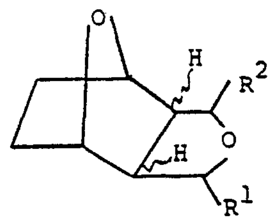

IV

(wherein one of $R^1$ and $R^2$ is benzyloxy and the other is H)
which is subjected to a hydrogenolysis reaction, for example, by reaction with hydrogen in the presence of palladium on charcoal to form the optically active hemiacetal

J

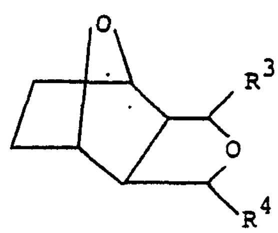

(wherein one of $R^3$ and $R^4$ is OH and the other is H).

As indicated, replacement of the *l*-menthol compound with the corresponding *d*-isomer in the reaction sequence produces, instead of the optically active (−) antipodes II or III or V or VI, the corresponding optically active (+) antipodes.

The hemiacetal *J* may then be employed to form optically active hemiacetal *L* by subjecting hemiacetal *J* to a homologation reaction, for example, by submitting *J* to Wittig reaction conditions by reacting *J* with an (alkoxymethyl)triphenylphosphonium halide such as (methoxymethyl)triphenylphosphonium chloride in the presence of an alkylamine like diisopropylamine, a lithium alkyl like n-butyl lithium in an inert organic medium like hexane, tetrahydrofuran or the like, at a temperature in the range of about −10° to 25°C to form optically active compound *K*

K

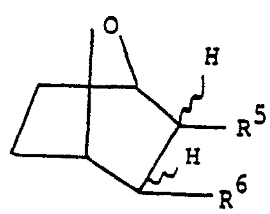

(wherein one of $R^5$ and $R^6$ is $CH_2OH$ and the other is $-CH{=}CH-OCH_3$).

Hydrolysis of alcohol compound *K* in the presence of strong acid such as trifluoroacetic acid produces optically active hemi-acetal *L* which is then subjected to Wittig reaction conditions and esterification, for example, reaction with a carboxyalkyl triphenylphosphonium halide, such as, triphenylcarboxybutylphosphonium chloride, in the presence of inert solvent, such as DMSO, to form optically an active alcohol

which is then reacted with, for example, a diazoalkane like diazomethane, to form the alcohol ester *M*

*M* 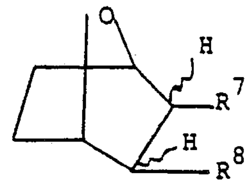

(wherein one of $R^7$ and $R^8$ is —$CH_2$—CH=CH— lower alkylene $CO_2CH_3$ and the other is —$CH_2OH$).

The optically active alcohol ester *M* may then be oxidized, for example, by reaction with chromium oxide with pyridine to form the optically active aldehyde *N*.

Thus, it is seen that the aldehyde *N* may be prepared from the starting hemiacetal without recourse to any chromatographic purification.

The prostaglandin aldehyde analog *N* may then be employed to prepare 7-oxabicycloheptane prostaglandins following the procedure as set out in US—A—4,143,054 to Sprague. Such prostaglandin derivatives are useful in the treatment of thrombolytic disease as explained in the above Sprague patent.

The wavy line (  ) which serves as a linking group for hydrogen in many of the formulae set out herein refers to the fact that the hydrogen atom may be cis exo or cis endo to the oxa bridge.

The nucleus in each of the compounds of the invention is depicted as

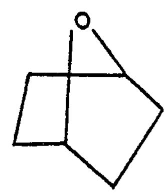

for matter of convenience; it will also be appreciated that the nucleus in the compounds of the invention may be depicted as

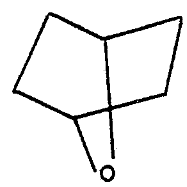

In carrying out the method of the invention, referring to the second reaction sequence described herein the racemic *cis-exo* hemiacetal

*G* 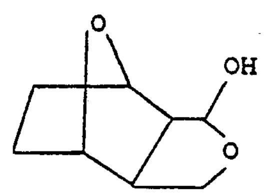

is converted to a diastereomeric mixture of menthol compounds *H* and *J* by reacting same with *l*-menthol in the presence of trace amounts of p-toluenesulfonic acid and an inert solvent such as benzene at reflux temperatures under an inert atmosphere, for example, nitrogen to form a mixture of diastereomers of formula *H* and *J* which is recrystallized, for example, from methanol and resolved to form the optically active acetal isomer *J*

*J* 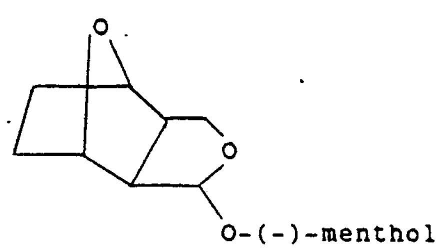

(90% optically pure).

The acetal *J* is allowed to react with benzyl alcohol at elevated temperatures of at least 110° to form the optically active benzyloxy compound

*K* 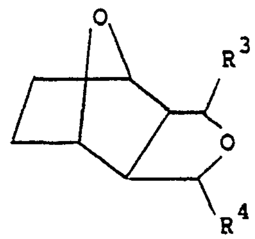

(wherein $R^4$ is benzyloxy and $R^3$ is H)
which is subjected to a hydrogenolysis reaction, for example, by reaction with hydrogen in the presence of palladium on charcoal to form the optically active hemiacetal

*L* 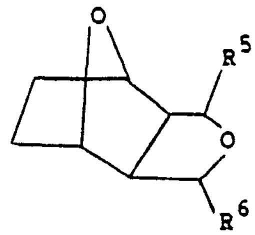

(wherein $R^6$ is OH and $R^5$ is H, 90% optically active).

The hemiacetal *L* (90% optically pure) is reacted with *l*-ketopinic acid halide, such as the chloride (prepared as described in US—A—4,235,891) employing a molar ratio of *L*:ketopinic acid chloride of about 0.5:1 to about 1:1, in the presence of 4-dimethylaminopyridine or (4-diethylaminopyridine and dicyclohexylcarbodiimide) and an inert solvent such as pyridine or dichloromethane to form a ketopinate ester II or III (where *l*-ketopinic acid halide is used).

The reaction sequence for preparing the optically active antipodes of compounds of formulae II and III, namely compounds of formulae IV and V, respectively, may be prepared following the above reaction sequence except that *d*-ketopinic acid halide is employed in place of the corresponding *l*-isomer.

The optically active ketopinate II (III) may then be subjected to ester hydrolysis, for example, by reacting the ketopinate with lithium hydroxide and hydrogen peroxide to form the optically active hemiacetal *M*

*M* 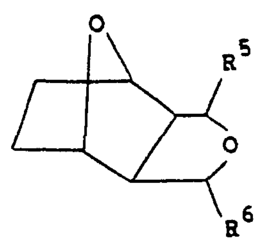

(wherein $R^6$ is OH and $R^5$ is H)
(100% optically active).

The hemiacetal *M* may then be employed to form optically active hemiacetal *O* by subjecting hemiacetal *M* to a homologation reaction, for example, by submitting *M* to Wittig reaction conditions by reacting *M* with an (alkoxymethyl)triphenylphosphonium halide such as (methoxymethyl)triphenylphosphonium chloride in the presence of an alkylamine like diisopropylamine, a lithium alkyl like n-butyl lithium in an inert organic medium like hexane, tetrahydrofuran or the like, at a temperature in the range of about −10° to 25°C to form optically active compound *N*

*N* 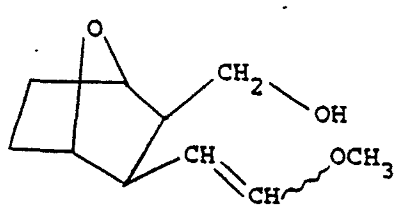

(wherein $R^7$ is $CH_2OH$ and $R^8$ is $—CH{=}CH—OCH_3$).

Hydrolysis of alcohol compound *N* in the presence of strong acid such as trifluoroacetic acid produces optically active hemiacetal *O* which is then subjected to Wittig reaction conditions and esterification, for

example, reaction with a carboxyalkyl triphenylphosphonium halide, such as, triphenylcarboxybutylphosphonium chloride, in the presence of inert solvent, such as DMSO, to form optically active alcohol which is then reacted with, for example, a diazoalkane like diazomethane, to form the alcohol ester *P*

*P* 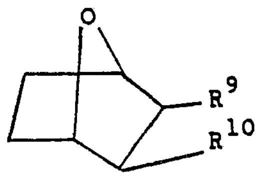

(wherein $R^{10}$ is $—CH_2—CH{=}CH$-lower alkylene $CO_2CH_3$ and $R^9$ is $—CH_2OH$).

The optically active alcohol ester *M* may then be oxidized, for example, by reaction with chromium oxide with pyridine to form the optically active aldehyde *O*.

Thus, it is seen that the aldehyde *O* may be prepared from the starting hemiacetal without recourse to any chromatographic purification.

The prostaglandin aldehyde analog *O* may then be employed to prepare 7-oxabicycloheptane prostaglandins following the procedure as set out in US—A—4,143,054 to Sprague. Such prostaglandin derivatives are useful in the treatment of thrombolytic disease as explained in the above Sprague patent.

The nucleus in each of the compounds of the invention is depicted as

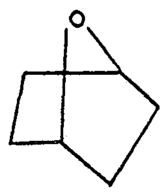

for matter of convenience; it will also be appreciated that the nucleus in the compounds of the invention may be depicted as

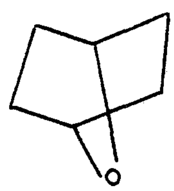

The following Examples represent preferred embodiments of the present invention.

## Examples 1 and 2

[3aS-[1-(1R,2S,5R),3aα,4α,7α,7aα]]-Octahydro-1-[[5-methyl-2-(1-methylethyl)-cyclohexyl]oxy]-4,7-epoxyisobenzofuran (Isomer I, Example 1) *and*
[3aR-[1-(1R,2S,5R),3aα,4α,7α,7aα]]-Octahydro-1-[[5-methyl-2-(1-methylethyl)cyclohexyl]oxy]-4,7-epoxyisobenzofuran (Isomer II, Example 2)

A solution of (exo)-octahydro-4,7-epoxyisobenzofuran-1-ol prepared as described in U.S. Patent No. 4,143,054 (21 g, 0.13 mole), levo-menthol (21 g, 0.13 mole) and p-toluenesulfonic acid (trace) in benzene (500 ml) was heated at reflux for 24 hours under nitrogen with a Dean-Stark trap containing molecular sieves in the system. The solution was chilled, washed with 5% sodium bicarbonate (200 ml), then concentrated *in vacuo*. The residue was recrystallized from methanol (300 ml) to yield 10 g of [3aR-[1-(1R,2S,5R),3aα,4α,7α,7aα]]-octhydro-1-[[5-methyl-2-(1-methylethyl)cyclohexyl]oxy]-4,7-epoxyisobenzofuran (pure isomer II), m.p. 109—111°C. The mother liquor was concentrated to 100 ml then treated with water to yield 10 g of solid which contained approximately 90% isomer I, that is [3aS-[1-(1R,2S,5R),3aα,4α,7α,7aα]]-octahydro-1-[[5-methyl-2-(1-methylethyl)cyclohexyl]oxy]-4,7-epoxyisobenzofuran and 10% isomer II by C—13 NMR. The mixture was recrystallized from pentane (100 ml) at −40°C to yield (after four recrystallizations) 8 g of product containing ~5% isomer II and 95% isomer I.

TLC: silica gel: ether/hexane; $R_f$=0.35; vanillin spray and heat.

## Examples 3 and 4

[3AS-(3aα,4α,7α,7aα)]-Octahydro-1-benzyloxy-4,7-epoxyisobenzofuran (Example 3 Isomer A) *and*
[3aR-(3aα,4α,7α,7aα)]-Octahydro-1-benzyloxy-4,7-epoxyisobenzofuran (Example 4 Isomer B)

A solution of isomer II [3aR-[1R,2S,5R),3aα,4α,7α,7aα]]-octahydro-1-[[5-methyl-2-(1-methylethyl)-cyclohexyl]oxy]-4,7-epoxy-isobenzofuran (from Example 2) (11.8 g, 0.04 mole) and p-toluene-sulfonic acid (trace) in benzyl alcohol (120 ml) was heated at 120°C under nitrogen for 4 hours. After this time, TLC (silica gel; ether/hexane (1:1)) indicated complete absence of starting material. The mixture was chilled, dissolved in ether, washed with 5% sodium bicarbonate and brine, dried over magnesium sulfate and concentrated

*in vacuo*. Excess benzyl alcohol was removed by sulfate and concentrated *in vacuo*. Excess benzyl alcohol was removed by distillation. The residue was purified by flash chromatography on LP—1 silica gel (700 ml) eluting with 20% and 50% ether/hexane mixtures to yield 750 mg of isomer A, an oil, and 7.8 g of isomer B, m.p. 47—50°C. These isomers appeared to be positional isomers of the benzyloxy group about the carbon atom.

TLC of isomer A: silica gel; hexane/ether (1:1),
$R_f$=0.25; vanillin spray and heat.
TLC of isomer B: silica gel; hexane/ether (1:1),
$R_f$=0.2; vanillin spray and heat.

Example 5

[1R-(1α,2β(5Z),3β,4α)]-7-[3-Formyl-7-oxabicyclo-[2.2.1]hept-2-yl]-5-heptenoic acid, methyl ester

A. *[3aS-(3aα,4α,7α,7aα)]-Octahydro-4,7-epoxyisobenzofuran-1-ol*

A mixture of isomer A (prepared as described in Example 3) (7.8 g, 0.032 mole), and 10% Pd/C (1 g) in ethyl acetate (250 ml) was stirred under one atmosphere of hydrogen until 707 ml of hydrogen had been consumed. The mixture was filtered and concentrated *in vacuo*. The residue was purified by flash chromatography with LP—1 silica gel (500 ml) eluting with ethyl acetate/dichloromethane (1:4) to yield 3.8 g of optically active title compound, m.p. 125°C.

$[\alpha]_D=-44°$ $[\alpha]^{Hg}_{365}=-122°$ c=10 mg/ml MeOH

TLC: silica gel; ethyl acetate/dichloromethane (1:1), $R_f$=0.2; vanillin spray and heat.

B. *[1R-(1α,2β,3β,4α]-1-(Hydroxymethyl-2-(2-methoxyethenyl)-7-oxabicyclo-[2.2.1]heptane*

A slurry of methoxymethyltriphenylphosphonium chloride (28.1 g, 0.082 mole) in toluene (700 ml) was treated with a solution of lithium diisopropylamide (prepared from 1.6 M n-butyl lithium (51 ml, 0.082 mole) and diisopropylamine (14.25 ml, 0.10 mole) in pentane) in tetrahydrofuran (20 ml). The mixture was stirred at room temperature for 30 minutes then treated with title A compound (3.7 g, 0.024 mole) dissolved in toluene (20 ml). The mixture was stirred at room temperature for 2 days. The reaction mixture was then poured into brine, acidified to pH=5 with concentrated hydrochloric acid, and extracted with ether (3×500 ml). The combined ether extracts were dried over magnesium sulfate and concentrated *in vacuo*. The residue was triturated with hexane/ether and filtered. The filtrate was concentrated *in vacuo* and the residue chromatographed on LP—1 silica gel (300 ml) eluting with pentane/ether (1:1) and ether to yield the desired title B product contaminated with phosphine oxide. This product was distilled *in vacuo* to yield 3 g of title B compound, b.p. 90°C/0.01 mm.

$[\alpha]_D=+44°$ $[\alpha]^{Hg}_{365}=+138°$ c=11 mg/ml MeOH

TLC: silica gel; ethyl acetate/dichloromethane (1:1);
$R_f$=0.2; vanillin spray and heat.

C. *[4aS-(4aα,5α,8α,8aα)]-Octahydro-5,8-epoxy-(1H)-benzopyran-3-ol*

A solution of title B compound (3 g, 0.016 mole) in 20% trifluoroacetic acid/water (30 ml) was stirred at room temperature under nitrogen for 2 hours. The solution was made basic with solid sodium bicarbonate. The aqueous solution was then saturated with sodium chloride and extracted with dichloromethane (6 × 200 ml). The combined extracts were concentrated *in vacuo*. The resultant oil contained significant amounts of partial hydrolysis products. This material was subjected to a second treatment with TFA as above and after a second workup as before yielded a solid which was recrystallized from cyclohexane to yield 2.4 g of title C compound, m.p. 104—105°C.

$[\alpha]_D=+27.2°$ $[\alpha]^{Hg}_{365}=0$ c = 7.9 mg/ml MeOH

D. *[1R-[1α,2β(5Z),3β,4α]]-7-[3-(Hydroxymethyl)-7-oxabicyclo[2.2.1]hept-2-yl]-5-heptenoic acid* and
E. *[1R-[1α,2β(5Z),3β,4α]]-7-[3-(Hydroxymethyl)-7-oxabicyclo[2.2.1]hept-2-yl]-5-heptenoic acid, methyl ester*

A slurry of 4-carboxybutyltriphenylphosphonium bromide (18.8 g, 0.0434 mole) in anhydrous dimethyl sulfoxide (36 ml) was treated with a solution of freshly prepared dimsyl ion at 15°C until an orange coloration persisted. A second equivalent of dimsyl ion was added to form the desired ylide. The deep red mixture was stirred at room temperature for 30 minutes then treated with title C compound (2.4 g, 0.0141 mole). The reaction mixture was stirred at room temperature for 2 hours then quenched with a solution of glacial acetic acid (2.58 g) in ether (10 ml). The mixture was poured into brine (1000 ml), acidified to pH=2 with concentrated hydrochloric acid and extracted with ethyl acetate (5×300 ml). The combined extracts were concentrated *in vacuo*. The residue was dissolved in 5% sodium bicarbonate and extracted with benzene (2×100 ml) and ethyl acetate (2×100 ml). The aqueous solution was then acidified to pH=2 with concentrated hydrochloric acid and extracted with ether (7×200 ml). The combined ether extracts were dried over magnesium sulfate and concentrated *in vacuo*. The residue was dissolved in ether (300 ml) and chilled overnight. The precipitated phosphine salts were removed by filtration. The filtrate containing title D acid was treated with excess diazomethane solution and stirred at room temperature for 1 hour. The reaction mixture was quenched with glacial acetic acid, washed with 5% sodium bicarbonate, then

concentrated *in vacuo.* The residue was purified by flash chromatography on LP—1 silica gel (600 ml) eluting with hexane/ether (1:1) and ether to yield 3 g of title E compound.

$[\alpha]_D=+11.2°$ $[\alpha]^{Hg}_{365}=0$ c = 16.9 mg/ml MeOH

TLC: silica gel; ether; $R_f=0.4$; vanillin spray and heat.

F. *[1R,[1α,2β(5Z),3β,4α]]-7-[3-Formyl-7-oxabicyclo[2.2.1]hept-2-yl]-5-heptenoic acid, methyl ester*

A solution of pyridine (10.75 ml, 0.133 mole) in anhydrous dichloromethane (350 ml) was treated portionwise with chromium trioxide (6.68 g, 0.067 mole) then stirred vigorously at room temperature for 30 minutes. The red mixture was then treated with dry celite (20 g) followed by title E compound (3 g, 0.0113 mole) dissolved in dichloromethane (20 ml). The mixture was stirred at room temperature for 30 minutes then filtered through celite. The filtrate was washed with 5% sodium bicarbonate (3 × 100 ml), 10% hydrochloric acid (2 × 250 ml), 5% sodium bicarbonate (1 × 200 ml), dried over magnesium sulfate, and concentrated *in vacuo.* The residue was dissolved in ether, treated with carbon and filtered. The filtrate was concentrated *in vacuo* to yield 2.9 g of title aldehyde.

TLC: silica gel; hexane/ether (1:1); $R_f=0.5$; vanillin spray and heat.

### Examples 6 and 7

[3aS-[1-(1R,2S,5R),3aα,4β,7β,7aα]]-Octahydro-1-[[5-methyl-2-(1-methylethyl)cyclohexyl]oxy]-4,7-epoxyisobenzofuran (Isomer I, Example 6) *and*
[3aR-[1R,2S,5R),3aα,4β,7β,7aα]]-Octahydro-1-[[5-methyl-2-(1-methylethyl)cyclohexyl]oxy]-4,7-epoxyisobenzofuran (Isomer II, Example 7)

A mixture of (endo)-octahydro-4,7-epoxyisobenzofuran-1-ol (prepared as described in U.S. Patent No. 4,143,054) (26.6 g, 0.17 mole), *l*-menthol (26.6 g, 0.17 mole) and p-toluenesulfonic acid (trace) was prepared in benzene (1400 ml). This mixture was heated at reflux for 18 hours under $N_2$ with a Dean-Stark trap containing molecular sieves in the system. The mixture was cooled, washed with 5% $NaHCO_3$ (500 ml) and concentrated yielding crystalline crude material. This was dissolved in hot MeOH (900 ml) and allowed to cool. Example 6 (Isomer I) compound (20.5 g) crystallized out first and concentration of the filtrate to 500 ml yielded an additional 2 g of Example 6 compound for a total of 22.5 g, m.p. 160—162°. isomer II (Example 7) was obtained by further concentrations of the mother liquor removing small amounts of mixed samples containing isomer I. When the percentage of I in the residue was >10% (as estimated by C—13 NMR) recrystallization of this from MeOH gave 17.8 g of pure Isomer II, m.p. 88—90°C.

### Example 8

[3aS-(3aα,4β,7β,7aα)]-Octahydro-1-benzyloxy-4,7-epoxyisobenzofuran

A mixture of Example 6 Isomer I compound (10 g, 0.034 mole) and p-toluenesulfonic acid (trace) was prepared in benzyl alcohol (100 ml) and heated at 120° for 4 hours. The mixture was cooled and partitioned between water and hexane. The hexane layer was washed with water (4 × 500 ml), treated with Norite, dried ($MgSO_4$) and concentrated to yield crude title compound which was recrystallized from EtOAc to yield 8.3 g of title benzyl acetal compound, m.p. 108—110°.

### Example 9

[1S-[1α,2α(5Z)3α,4α]]-7-[3-Formyl-7-oxabicyclo[2.2.1]hept-2-yl]-5-heptenoic acid, methyl ester

A. *[3aS-(3aα,4β,7β,7aα)]-Octahydro-4,7-epoxyisobenzofuran-1-ol*

A mixture of Example 8 compound (18.03 g, 73.3 mmole) and 10% palladium on charcoal (1.8 g) was prepared in ethyl acetate (200 ml) and hydrogenated at atmospheric pressure until 1 mole of $H_2$ had been consumed (~1 hour, 1641 ml). The catalyst was removed by filtration and, after concentration of the filtrate, 11.5 g of crystalline title hemiacetal m.p. 120—120° was obtained. Recrystallization from cyclohexane-benzene yielded 10.2 g of title A compound, m.p. 132° $[\alpha]_D = -79°$. C=10 mg/ml, MeOH.

B. *[1S-(1α,2α,3α,4α)-2-(2-Methoxyethenyl)-3-hydroxymethyl-7-oxabicyclo[2.2.1]heptene*

Lithium diisopropylamide (from 96 mmole of n-butyl Li (1.6 mmole/ml) in hexane and 120 mmole of diisopropylamine (16.8 ml)) was prepared under anhydrous conditions and dissolved in dry THF (20 ml). This was added to a mixture of triphenylmethoxymethylphosphonium chloride (33 g, 96 mmol) and dry toluene (450 ml) at 0° under argon with mechanical stirring. After stirring the resulting red ylide (nearly everything dissolved) for 30 minutes at 0°, the title A hemiacetal (5.0 g, 32 mmole) in powdered form was added via a solids addition device and the resulting mixture stirred at 20° for 5 hours. The reaction was quenched by addition of HOAc (96 mmole) and poured into brine (1 L). This was extracted with ether (3 × 300 ml), the extracts dried ($MgSO_4$) and concentrated to yield an oil. Purification by flash chromatography on LP-1 silica gel (500 ml) eluting with 50/50 ether/pentane to start and finishing with ether gave 4.8 g of enolether which on distillation (b.p. 107—112° @ 0.001 mm) gave 3.8 g of pure enolether containing ~5% (by NMR) unchanged title B compound.

C. *[4aS-(4aα,5β,8β,8aα)]-Octahydro-5,8-epoxy-(1H)-benzopyran-3-ol*

A mixture of crude title B compound (containing ~5% title B compound) (6.1 g) and trifluoroacetic acid

(20% in $H_2O$, 100 ml) was prepared and rapidly stirred under argon at 25° for 2 hours. The acid was decomposed with solid $NaHCO_3$, and the neutral mixture saturated with salt and extracted with $CH_2Cl_2$ (6 × 300 ml). The extracts were dried ($MgSO_4$) and concentrated to yield an oil which contains significant amounts of partial hydrolysis products. This material was subjected to a second treatment with TFA as above and after a second work up yielded 5.7 g of product which consisted of title C compound contaminated with title B compound. These two materials were separated by flash chromatography on LP-1 silica gel (600 ml) using 70/30 ether/pentane as the eluent. Yield of pure title C compound 3.75 g $[\alpha]_D$ = +15°. C=8.3 mg/ml, MeOH.

D. *[1S-[1α,2α(5Z),3α,4α]]-7-[3-(Hydroxymethyl)-7-oxabicyclo[2.2.1]hept-2-yl]-5-heptenoic acid* and
E. *[1S-[1α,2α(5Z),3α,4α]]-7-[3-(Hydroxymethyl)-7-oxabicyclo[2.2.1]hept-2-yl]-5-heptenoic acid, methyl ester*

A mixture of triphenylcarboxybutylphosphonium ylide was prepared in dry DMSO (60 ml) from triphenylcarboxybutylphosphonium bromide (29.3 g, 66 mmole) and freshly prepared dimsyl ion at 25° under argon. To this was added title C compound (3.75 g, 22 mmole) dissolved in DMSO (10 ml) and the resulting mixture stirred at 25° for 2 hours. The mixture was quenched by HOAc (66 mmole) in ether (5 ml) and then poured into brine (1 L) and extracted with EtOAc (4 × 300 ml). The extracts were dried ($MgSO_4$) and concentrated to yield an oil which was shaken with saturated $NaHCO_3$ to separate acidic products from triphenylphosphine oxide. This mixture was extracted with benzene (3 × 100 ml) followed by EtOAc (3 × 100 ml) then acidified to pH 2 with concentrated HCl and extracted with ether (6 × 200 ml). The combined extracts were dried ($MgSO_4$) and chilled whereupon a crystalline material appeared. This was removed after 24 hours and the filtrate concentrated to yield 4.0 g of crude acid title D acid. The acid was esterified directly by treatment in ether with excess diazomethane at 25°. Title E ester was purified by flash column chromatography on LP-1 silica gel (500 ml) eluting with 50/50 hexane/ether followed by ether to yield 2.91 g of title E ester $[\alpha]_{Hg,365}$ = −23° (C—12.5 mg, MeOH. (TLC $R_f$ = 0.6, silica gel EM, ether, spot visualization with PMA + $H^{\oplus}$ + Δ).

F. *[1S-[1α,2α(5Z),3α,4α]]-7-[3-Formyl-7-oxabicyclo[2.2.1]hept-2-yl]-5-heptenoic acid, methyl ester*

A mixture of chromium oxide (6.48 g, 6.48 mmole), pyridine (10.4 ml, 129.6 mmole) and $CH_2Cl_2$ (340 ml) was prepared and stirred at 25° for 30 minutes under argon. Dry celite (18 g) was added followed by title E ester (2.91 g, 10.8 mmole) dissolved in $CH_2Cl_2$ (15 ml) and stirring was continued for 30 minutes. The mixture was filterd and the filtrate washed with 5% $NaHCO_3$ (3 × 100 ml), 5% $NaHSO_4$ (2 × 250 ml), 5% $NaHCO_3$ (1 × 200 ml), dried ($MgSO_4$) and concentrated. The residue was dissolved in ether, treated with Norite, filtered and concentrated to yield 2.6 g of title aldehyde.

Examples 10 and 11

[3aS-[1-(1S,2R,5S),3aα,4α,7α,7aα]]-Octahydro-1-[[5-methyl-2-(1-methylethyl)cyclohexyl]oxy]-4,7-epoxyisobenzofuran (Example 10, Isomer I) and
[3aR-[1-(1S,2R,5S),3aα,4α,7α,7aα]]-Octahydro-1-[[5-methyl-2-(1-methylethyl)cyclohexyl]oxy]-4,7-epoxyisobenzofuran (Example 11, Isomer II)

Following the procedure of Examples 1 and 2 except substituting *d*-menthol for *l*-menthol, the title compounds are obtained.

Examples 12 and 13

[1aR-(3aα,4α,7α,7aα)]-Octahydro-1-benzyloxy-4,7-epoxyisobenzofuran (Example 12, Isomer A) and
[3aS-[3aα,4α,7α,7aα)]-Octahydro-1-benzyloxy-4,7-epoxyisobenzofuran (Example 13, Isomer B)

Following the procedure of Examples 3 and 4 except substituting the Example 11, Isomer II compound for the Example 2, Isomer II compound, the title compounds are obtained.

Example 14

[3aR-(3aα,4α,7α,7aα)]-7-[3-Formyl-7-oxabicyclo[2.2.1]hept-2-yl]-5-heptenoic acid, methyl ester

Following the procedure of Example 5 except substituting the Example 12, Isomer A compound for the Example 3, Isomer A compound, the title aldehyde is obtained.

Example 15 and 16

[3aS-[1-(1S,2R,5S),3aα,4β,7β,7aα]]-Octahydro-1-[[5-methyl-2-(1-methylethyl)cyclohexyl]oxy]-4,7-epoxyisobenzofuran (Example 15, Isomer I) *and*
[3aR-[1-(1S,2R,5S),3aα,4β,7β,7aα]]-Octahydro-1-[[5-methyl-2-(1-methylethyl)cyclohexyl]oxy]-4,7-epoxyisobenzofuran (Example 16, Isomer II)

Following the procedure of Examples 6 and 7 except substituting *d*-menthol for *l*-menthol, the title compound are obtained.

Example 17

[3aR-(3aα,4β,7β,7aα]]-Octahydro-1-benzyloxy-4,7-epoxyisobenzofuran

Following the procedure of Example 8 except substituting the Example 16, Isomer II compound for the Example 6, Isomer I compound, the title compound is obtained.

Example 18

[1R-[1α,2α(5Z),3α,4α]]-7-[3-Formyl-7-oxabicyclo[2.2.1]hept-2-yl]-5-heptenoic acid, methyl ester

Following the procedure of Example 9 except substituting the Example 17 compound for the Example 8 compound, the title compound is obtained.

Example 19

[1R-[1α(1S),3aα,4α,7α,7aα]]-Octahydro-1-[[2-oxo-7,7-dimethylbicyclo[2.2.1]heptane]carboxyl]-4,7-epoxyisobenzofuran

A. *[1S-[1α(1R,2S,5R),3aα,4α,7aα]]-Octahydro-1-[[5-methyl-2-(1-methylethyl)-cyclohexyl]oxy]-4,7-epoxyisobenzofuran* and

B. *[1R-[1α(1R,2S,5R),3aα,4α,7α,7aα]]-Octahydro-1-[[5-methyl-2-(1-methylethyl)-cyclohexyl]oxy]-4,7-epoxyisobenzofuran*

A solution of (exo)-octahydro-4,7-epoxyisobenzofuran-1-ol (prepared as described in U.S. Patent No. 4,143,054) (30 g, 0.192 mole), levo-menthol (30 g, 0.192 mole) and *p*-toluene-sulfonic acid (100 mg) in benzene (500 ml) was heated at reflux for 24 hours under nitrogen with a Dean-Stark trap containing molecular sieves in the system. The solution was chilled, washed with 5% sodium bicarbonate (100 ml), then concentrated *in vacuo*. The residue was recrystallized from methanol (300 ml) to yield 15 g of pure isomer A, m.p. 109—111°C. The mother liquor was concentrated *in vacuo*. The residue was chromatographed on LP-1 silica gel (600 ml) eluting with hexane and ether/hexane (1:9) to yield 20 g of isomeric mixture A and B. This mixture was recrystallized from methanol (150 ml) to yield 7 g of pure isomer A (100% optically pure), m.p. 109—111°C. The mother liquor was concentrated to yield 24 g of isomer B which was approximately 90% optically pure by C-13 NMR.

C. *[1S,3aR-(1α,3aα,4α,7α,7aα)]-Octahydro-1-benzoyloxy-4,7-epoxyisobenzofuran*

A solution of isomer B (prepared as described above) (24 g, 0.082 mole) and *p*-toluenesulfonic acid (100 mg) in benzyl alcohol (250 ml) was heated at 90°C for 90 minutes. TLC (silica gel; ether/hexane (1:1)) indicated complete absence of starting material. The mixture was chilled, diluted with ether (1000 ml), washed with 5% sodium bicarbonate, then concentrated *in vacuo*. Excess benzyl alcohol was removed by distillation. The residue was dissolved in hexane (200 ml) and chilled overnight to yield 13 g of title C compound, an oil at room temperature.

TLC: silica gel; hexane/ether (1:1), $R_f$ = 0.2; vanillin spray and heat.

D. *[3aR-(3aα,4α,7α,7aα)]-Octahydro-4,7-epoxyisobenzofuran-1-ol*

A solution of title C compound (13 g, 0.053 mole) and trifluoroacetic acid (40 ml) in water (160 ml) was stirred at room temperature under nitrogen for 4 hours. The mixture was neutralized with solid sodium bicarbonate, saturated with sodium chloride and extracted with dichloromethane (8 × 200 ml). The combined extracts were dried over magnesium sulfate and concentrated *in vacuo*. The residue was chromatographed on LP-1 silica gel (500 ml) eluting with ethyl acetate/dichloromethane (2:5) to yield 8 g of title D compound (90% optically pure).

TLC: silica gel; ethyl acetate/dichloromethane (1:1); $R_f$ = 0.2; vanillin spray and heat.

*E. [1R-[1α(1S),3aα,4α,7α,7aα]]-Octahydro-1-[[2-oxo-7,7-dimethylbicyclo[2.2.1]heptane]carboxyl]-4,7-epoxyisobenzofuran*

A solution of title D compound (8.75 g, 0.056 mole), levo-ketopinic acid (Org. Syn., *45,* p. 55) (10.21 g, 0.056 mole), 4-dimethylaminopyridine (6.84 g, 0.56 mole) and dicyclohexylcarbodiimide (11.55 g, 0.056 mole) in dichloromethane (200 ml) was stirred at room temperature under nitrogen for 4 days. The mixture was filtered and the filtrate washed with 5% potassium bisulfate (2 × 100 ml), 5% sodium bicarbonate (2 × 100 ml) and water. The organic layer was dried over magnesium sulfate and concentrated *in vacuo*. The residue was recrystallized 3 times from diisopropyl ether to yield 10 g of title compound, m.p. 148—150°C.

TLC: silica gel; ether/hexane (1:1), $R_f$ = 0.2; vanillin spray and heat.

$[\alpha]_D$ = +48°C = 20 mg/ml ($CHCl_3$).

Example 20

[1S-(1α,2β(Z),3β,4α)]-7-[3-Hydroxymethyl)-7-oxabicyclo[2.2.1]hept-2-yl]-5-heptenoic acid, methylester

A. *[3aR-(1α,3aα,4α,7α,7aα)]-Octahydro-4,7-epoxyisobenzofuran-1-ol*

A solution of Example 19 compound (8.8 g, 0.0275 mole) in tetrahydrofuran (150 ml) was treated with 1N sodium hydroxide (55 ml, 0.055 mole) and stirred at room temperature for 4 hours. The reaction mixture was treated with 1N hydrochloric acid (55 ml, 0.055 mole), saturated with sodium chloride, and extracted with chloroform (10 × 100 ml). The combined extracts were dried over magnesium sulfate and concentrated *in vacuo*. The residue was chromatographed on LP-1 silica gel (500 ml) eluting with

dichloromethane and ethyl acetate/dichloromethane (1:1) to yield 4 g of product which was recrystallized from benzene/cyclohexane to yield 3.7 g of title A compound, m.p. 65—67°C.

TLC: silica gel; ethyl acetate/dichloromethane (1:1); $R_f$ = 0.2; vanillin spray and heat.

$[\alpha]_D$ = +46°C = 20 mg/ml ($CHCl_3$).

B. *[(1R-(1α,2β,3β,4α)]-3-(2-Methoxyethenyl)-7-oxabicyclo[2.2.1]heptane-2-methanol*

A slurry of methoxymethyltriphenylphosphonium chloride (15.2 g, 0.0443 mole) in toluene (500 ml) was treated with a solution of lithium diisopropylamide (prepared from 1.6 M n-butyl lithium (27.6 ml, 0.0442 mole) and diisopropylamine (7.7 ml, 0.055 mole) in pentane in tetrahydrofuran (10 ml) at 0°C. The mixture was stirred at room temperature for 30 minutes then treated *via* a solid addition device with title A compound (2 g, 0.0128 mole). The mixture was stirred at room temperature for 3 days then poured into brine (1000 ml), treated with 10% hydrochloric acid until pH = 6.5, then extracted with ether (3 × 200 ml). The combined ether extracts were dried over magnesium sulfate and concentrated *in vacuo*. The residue was triturated with hexane/ether and filtered. The filtrate was concentrated *in vacuo*. The residue was chromatographed on LP-1 silica gel (300 ml) eluting with dichloromethane and ether/dichloromethane (1:1) to yield the desired product contaminated with phosphine salts. This material was distilled *in vacuo* to yield 1.6 g of title B compound, b.p. 110°C/0.05 cm.

$[\alpha]_D$ = −54° $[\alpha]^{Hg}_{365}$ = −168°C = 11 mg/ml $CHCl_3$

TLC: silica gel; ethyl acetate/dichloromethane (1:1); $R_f$ = 0.2; vanillin spray and heat.

C. *[(4aR)-cis-exo]-Octahydro-5,8-epoxy-1H-benzopyran-3-ol*

A solution of title B compound (1.6 g, 0.0087 mole) in 20% trifluoroacetic acid/water (16 ml) was stirred at room temperature under nitrogen for 2 hours. The solution was made basic with solid sodium bicarbonate. The aqueous mixture was then saturated with sodium chloride and extracted with chloroform (10 × 50 ml). The combined extracts were concentrated *in vacuo*. The resultant oil contained significant amounts of partial hydrolysis products. This material was subjected to a second treatment with TFA/water as above and after a second workup as above yielded a solid product which was chromatographed on LP-1 silica gel (150 ml) eluting with ether/dichloromethane (1:9) and ether to yield 1.3 g of title C compound, m.p. 94—96°C.

TLC: silica gel; ethyl acetate/dichloromethane (1:1); $R_f$ = 0.1; vanillin spray and heat.

$[\alpha]_D$ = −26.8°C = 10 mg/ml MeOH.

D. *[1S-(1α,2β(Z),3β,4α)]-7-[3-(Hydroxymethyl)-7-oxabicyclo-[2.2.1]hept-2-yl]-5-heptenoic acid* and
E. *[1S-(1α,2β(Z),3β,4α)]-7-[3-(Hydroxymethyl)-7-oxabicyclo[2.2.1]hept-2-yl]-5-heptenoic acid, methyl ester*

A slurry of 4-carboxybutyltriphenylphosphonium bromide (10.18 g, 0.023 mole) in anhydrous dimethylsulfoxide (20 ml) was treated with a solution of freshly prepared dimsyl ion at 15°C until an orange coloration persisted. A second equivalent of dimsyl ion was added to form the desired ylide. The deep red mixture was stirred at room temperature for 30 minutes then treated with title compound (1.3 g, 0.0076 mole) dissolved in 10 ml of anhydrous dimethylsulfoxide. The reaction mixture was stirred at room temperature for 2 hours then quenched with a solution of glacial acetic acid (1.4 g, 0.023 mole) in ether (10 ml). The mixture was poured into brine (1000 ml), acidified to pH = 2 with concentrated hydrochloric acid and extracted with ethyl acetate (6 × 50 ml). The combined extracts were concentrated *in vacuo*. The residue was dissolved in 5% sodium bicarbonate (150 ml) and extracted with benzene (2 × 100 ml) and ethyl acetate (2 × 100 ml). The aqueous layer was acidified to pH = 2 with concentrated hydrochloric acid and extracted with ethyl acetate (5 × 50 ml). The combined ethyl acetate extracts were dried over magnesium sulfate and concentrated *in vacuo*. The residue was dissolved in ether (100 ml) and chilled overnight. The precipitated phosphine salts were removed by filtration. The filtrate was concentrated *in vacuo* to yield 2.1 g of product which contained title D compound contaminated with small amounts of phosphine salts. Title D compound was dissolved in 100 ml of ether, treated with excess ethereal diazomethane solution and stirred at room temperature for 1 hour. The reaction mixture was quenched with glacial acetic acid/ether solution, washed with 5% sodium bicarbonate, the concentrated *in vacuo*. The residue was chromatographed on LP-1 silica gel (400 ml) eluting with ether/hexane (1:1) and ether to yield 1.4 g of title E compound.

TLC of title D compound: silica gel: ether: $R_f$=0.2; vanillin spray and heat.

TLC of title E compound: silica gel: ether: $R_f$=0.4; vanillin spray and heat.

$[\alpha]_D$ of title E compound= −12.3° C=27 mg/ml (MeOH).

## Example 21

[1S-[1α(1S),3aα,4α,7α,7aα]]Octahydro-1-[[2-oxo-7,7-dimethylbicyclo[2.2.1]-heptane]-carboxyl]-4,7-epoxyisobenzofuran

Following the procedure of Example 19 except substituting dextro-menthol for levo-menthol and dextro-ketopinic acid for levo-ketopinic acid, the title compound is obtained.

Example 22

[1R-[(1α,2β(Z),3β,4α)]-7-[3-(Hydroxymethyl)-7-oxabicyclo[2.2.1]hept-2-yl]-5-heptenoic acid, methylester

Following the procedure of Example 20 except substituting the Example 21 isomer for the Example 19B isomer, the title compound is obtained.

Examples 23 and 24

[1S-[1α(1S),3aα,4β,7β,7aα]]1,3,3a,4,7,7a-Hexahydro-1-[[2-oxo-7,7-dimethylbicyclo[2.2.1]-heptane]carboxyl-4,7-epoxyisobenzofuran (Example 23) and [1R-[1α(1S),3aα,4β,7aα]]-1,3,3a,4,7,7a-Hexahydro-1-[[2-oxo-7,7-dimethylbicyclo[2.2.1]heptane]carboxyl]-4,7-epoxyisobenzofuran (Example 24)

A solution of (endo)-1,3,3a,4,7,7a-hexahydro-4,7-epoxyisobenzofuran-1-ol (prepared as described in Example 14 of US—A—4,143,054) (53.2 g, 343 mmoles), *l*-ketopinic acid chloride (70 g, 350 mmoles) and 4-dimethylaminopyridine (1 g, 8 mmoles) in pyridine (500 ml) was stirred at room temperature for 18 hours. The reaction mixture was concentrated *in vacuo*. The residue was diluted with water and extracted with dichloromethane (3 × 300 ml). The combined extracts were dried over magnesium sulfate and concentrated *in vacuo* and found to contain a mixture of Examples 23 and 24 isomers. The crude product was recrystallized 4 times from isopropyl ether to yield 19 g of title Example 23 product, m.p. 123—125°C.

$[\alpha]_D$ = +58°C = 1 $CHCl_3$.

TLC: silica gel; ether/hexane (1:1); $R_f$ = 0.2; vanillin spray + heat.

Example 25

[1R-(1α,2α,3α,4α)]-3-(Acetoxymethyl)-7-oxabicyclo[2.2.1]heptane-2-carboxaldehyde

A. *[1S-[3aα,4β,7β,7aα]]-1,3,3a,4,7,7a-Hexahydro-4,7-epoxyisobenzofuran-1-ol*

A solution of Example 23 compound (6.36 g, 20 mmole) in tetrahydrofuran (100 ml) was treated with 1 normal lithium hydroxide (20 ml, 20 mmole) and 30% hydrogen peroxide (0.23 ml, 20 mmole) then stirred vigorously in a Morton flask at room temperature for 2 hours. TLC after this time indicated complete absence of starting material. The solution was quenched with 5% sodium thiosulfate (5 ml) then saturated with sodium chloride and extracted with chloroform (10 × 50 ml). The combined extracts were dried over magnesium sulfate and concentrated. The residue was recrystallized from benzene/cyclohexane to yield 3 g of desired hemiacetal, m.p. 99—100°C.

TLC: silica gel; ethyl acetate/dichloromethane (1:1); $R_f$ = 0.2; vanillin spray + heat.

$[\alpha]_D$ = +67°C = 10 mg/ml $CHCl_3$.

B. *[1R-(1α,2α,3α,4α)]-3-(Hydroxymethyl)-7-oxa-5-bicyclo[2.2.1]heptene-2-carboxaldehyde 1,1-dimethylhydrazone*

A solution of title B hemiacetal (2.8 g, 18 mmole) and unsym-dimethylhydrazine (2.2 g, 37 mmole) in dichloromethane (50 ml) was stirred at room temperature overnight. The reaction mixture was then concentrated *in vacuo* to yield 3.5 g of desired title B product.

TLC: silica gel; ether; $R_f$ = 0.15; vanillin spray + heat.

C. *[1R-(1α,2α,3α,4α)]-3-(Acetoxymethyl)-7-oxa-5-bicyclo[2.2.1]heptene-2-carboxaldehyde 1,1-dimethylhydrazone*

A solution of title B compound (11.5 g, 59 mmole), trimethylamine (61 g, 600 mmoles) and 4-dimethylaminopyridine (0.465 g, 3.8 mmoles) in acetic anhydride (31 g, 300 mmoles) was stirred at room temperature for 2 hours. After this time TLC indicated complete conversion to the acetate. The mixture was concentrated *in vacuo*. The residue was poured into ice water and extracted with methylene chloride. The organic layer was dried over magnesium sulfate and concentrated *in vacuo*. The residue was chromatographed on LP-1 silica (1500 ml) eluting with ether/hexane (1:1) to yield 14 g of title C compound.

TLC: silica gel; ether; $R_f$ = 0.4; vanillin spray + heat.

D. *[1R-(1α,2α,3α,4α)]-3-(Acetoxymethyl)-7-oxabicyclo[2.2.1]heptane-2-carboxaldehyde 1,1-dimethylhydrazone*

A solution of title C compound (2.1 g, 8.8 mmole) in ethyl acetate (100 ml) was treated with 10% Pd/C (200 mg) and stirred under one atmosphere of hydrogen until 200 ml of hydrogen had been consumed. The mixture was filtered through celite and concentrated to yield 2.1 g of title D compound. CMR indicated that the product still contained 5% of the undesired *trans* isomer.

TLC: silica gel; ether; $R_f$ = 0.4; vanillin spray + heat.

E. *[1R-(1α,2α,3α,4α)]-3-(Acetoxymethyl)-7-oxabicyclo[2.2.1]heptane-2-carboxaldehyde*

A solution of title D compound (2.1 g, 8.8 mmole) in tetrahydrofuran (130 ml) was treated with a solution of cupric chloride dihydrate (2.98 g, 17.6 mmole) in pH = 7 phosphate buffer (140 ml). The mixture was stirred at room temperature for 2 hours, then concentrated *in vacuo* to approximately 150 ml. The mixture was diluted with dichloromethane (300 ml) and filtered through celite. The filtrate was washed with

5% sodium bicarbonate, dried over magnesium sulfate, and concentrated *in vacuo* to produce title aldehyde.

Examples 26 and 27

[1S-[1α(1R),3aα,4β,7β,7aα]]-1,3,3a,4,7,7a-Hexahydro-1-[[2-oxo-7,7-dimethylbicyclo[2.2.1]heptane]carboxyl]-4,7-epoxyisobenzofuran (Example 26) and [1R-[1α(1R),3aα,4β,7β,7aα]]-1,3,3a,4,7,7a-Hexahydro-1-[[2-oxo-7,7-dimethylbicyclo[2.2.1]heptane]carboxyl]-4,7-epoxyisobenzofuran (Example 27)

A solution of (endo)-1,3,3a,4,7,7a-hexahydro-4,7-epoxyisobenzofuran-1-ol (12.7 g, 82.5 mmole) *d*-ketopinic acid (15 g, 82 mmole), and 4-dimethylaminopyridine (10.1 g, 82 mmole) in anhydrous dichloromethane (400 ml) was treated with dicyclohexylcarbodiimide (DCC) (17.1 g, 82 mmole) and stirred at room temperature for 3 days. The reaction mixture was then filtered. The filtrate was washed with 5% potassium bisulfate, 5% sodium bicarbonate and water. The dichloromethane solution was dried over magnesium sulfate and concentrated *in vacuo*. The residue was chromatographed on LP-1 silica gel (600 ml) eluting with dichloromethane and ether/dichloromethane (1:4). Fractions containing the desired ester (Example 26) and Example 27 ester were combined and concentrated *in vacuo*. The solid residue was recrystallized 3 times from isopropyl ether to yield 5.5 g of optically active title Example 26 compound, m.p. 135—138°C.

$[\alpha]_D = -57°C = 10$ mg/ml $CHCl_3$.

TLC: silica gel; ether/hexane (1:1), $R_f = 0.2$; vanillin spray + heat.

Example 28

[1S-(3aα,4β,7β,7aα)]-1,3,3a,4,7,7a-Hexahydro-4,7-epoxyisobenzofuran-1-ol

A solution of Example 26 compound (5.5 g, 17.3 mmole) in tetrahydrofuran (90 ml) was treated with 1N lithium hydroxide solution (17.3 ml, 17.3 mmole) and 30% hydrogen peroxide (0.2 ml, 17.3 mmole) then stirred vigorously in a Morton flask at room temperature for 2 hours. After this time, TLC indicated complete absence of starting material. The solution was quenched with 5% sodium thiosulfate solution (5 ml), saturated with sodium chloride, and extracted with chloroform (10 × 50 ml). The combined extracts were dried over magnesium sulfate and concentrated *in vacuo*. The residue was recrystallized from benzene to yield 2 g of title compound, m.p. 105—109°C.

TLC: silcia gel: ethyl acetate/dichloromethane (1:1); $R_f = 0.2$; vanillin spray + heat.

$[\alpha]_D = -67°C = 10$ mg/ml $CHCl_3$.

B. *[1S-(1α,2α,3α,4α)]-3-(Hydroxymethyl)-7-oxa-5-bicyclo[2.2.1]heptene-2-carboxaldehyde 1,1-dimethylhydrazone*

A solution of title A compound (2 g, 13 mmole) and unsymdimethylhydrazine (1.6 g, 27 mmole) in dichloromethane (75 ml) was stirred overnight at room temperature. The reaction mixture was then concentrated *in vacuo* to yield 2.5 g of title B compound.

TLC: silica gel; ether; $R_f = 0.15$; vanillin spray + heat.

C. *[1S-(1α,2α,3α,4α)]-3-(Acetoxymethyl)-7-oxa-5-bicyclo[2.2.1]heptene-2-carboxaldehyde 1-,1-dimethylhydrazone*

A solution of title B compound (2.5 g, 12.7 mmole) in acetic anhydride (15 ml) was stirred at room temperature for 8 hours then concentrated *in vacuo*. The residue was dissolved in ether and washed with 5% sodium bicarbonate and brine. The ether layer was chromatographed on LP-1 silica gel (500 ml) eluting with ether/pentane (1:1) to yield 775 mg of title C compound.

TLC: silica gel; ether; $R_f = 0.4$; vanillin spray + heat.

D. *[1S-(1α,2α,3α,4α)]-4-(Acetoxymethyl)-7-oxabicyclo[2.2.1]heptene-2-carboxaldehyde 1,1-dimethylhydrazone*

A solution of title C compound (775 mg, 3.26 mmole) in ethyl acetate (100 ml) was treated with 10% Pd/C (100 mg) and stirred under one atmosphere of hydrogen until 75 ml of hydrogen had been consumed. The mixture was filtered and concentrated *in vacuo* to yield 775 mg of title D compound.

TLC: silica gel; ether; $R_f = 0.4$; vanillin spray + heat.

E. *[1S-(1α,2α,3α,4α)]-3-(Acetoxymethyl)-7-oxabicyclo[2.2.1]heptane-2-carboxaldehyde*

A solution of title D compound (775 mg, 3.2 mmole) in tetrahydrofuran (48 ml) was treated with a solution of cupric chloride dihydrate (1.1 g, 6.5 mmole) in pH = 7 phosphate buffer (52 ml). The mixture was stirred at room temperature for 2 hours, then concentrated *in vacuo* to approximately one-half its original volume. The mixture was diluted with dichloromethane (100 ml) and filtered through celite. The filtrate was washed with 5% sodium bicarbonate, dried over magnesium sulfate and concentrated *in vacuo* to give title product.

**Claims for the Contracting States: BE CH DE FR GB IT LI LU NL SE**

1. A compound having the structure 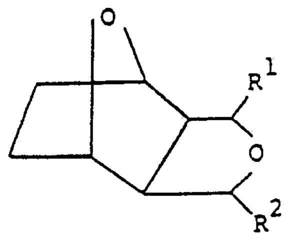

wherein one of $R^1$ and $R^2$ is 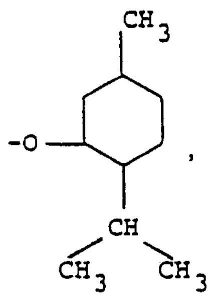, 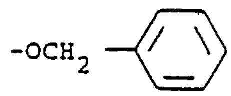,

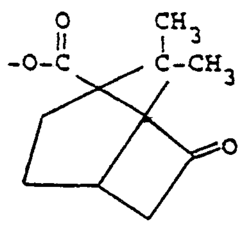 or 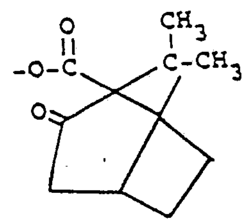

and the other is hydrogen, or having the structure 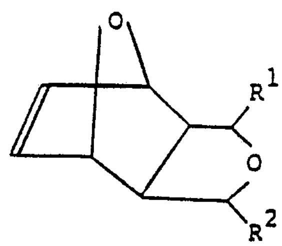

wherein one of $R^1$ and $R^2$ is 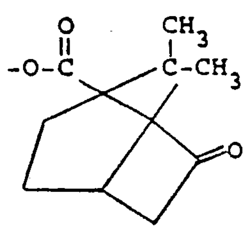 or 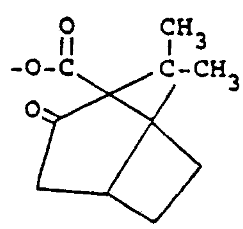

and the other is hydrogen.

2. The compound as defined in Claim 1 wherein $R^1$ is H and $R^{21}$ is

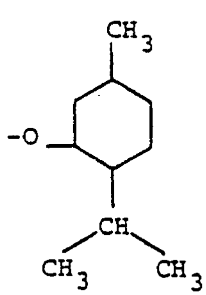 or 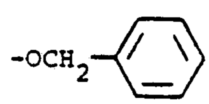.

3. The compound as defined in Claim 1 wherein $R^2$ is H and $R^1$ is

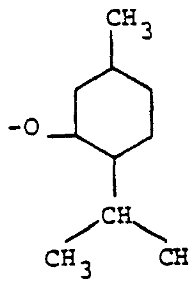

or

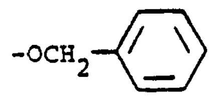

.

4. The compound as defined in Claim 1 having the formula

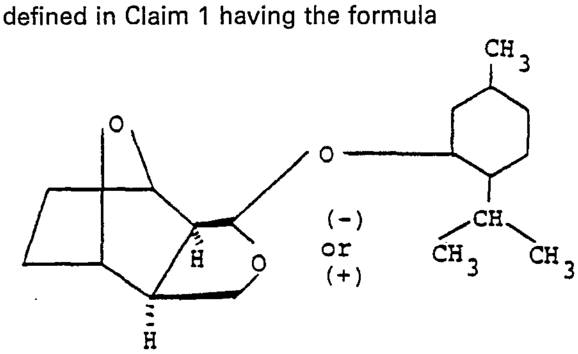

(-)
or
(+)

.

5. The compound as defined in Claim 1 having the formula

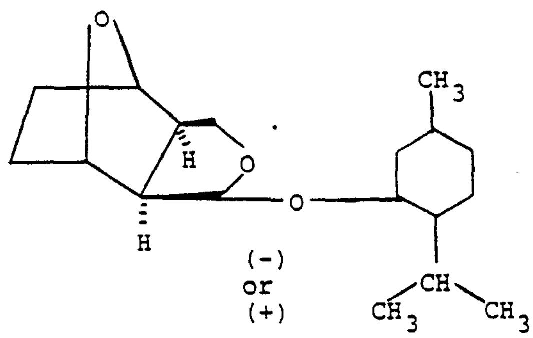

(-)
or
(+)

.

6. The compound as defined in Claim 1 having the formula

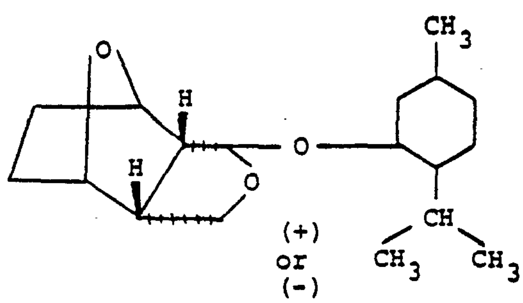

(+)
or
(-)

.

7. The compound as defined in Claim 1 having the formula

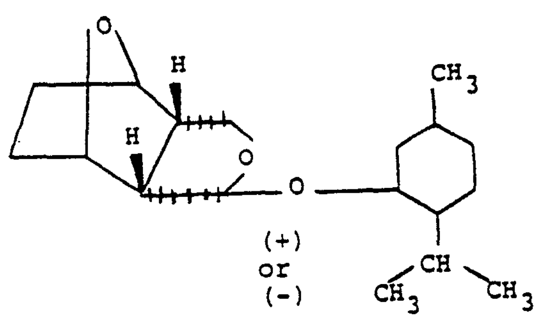

(+)
or
(-)

.

8. The compound as defined in Claim 1 having the formula

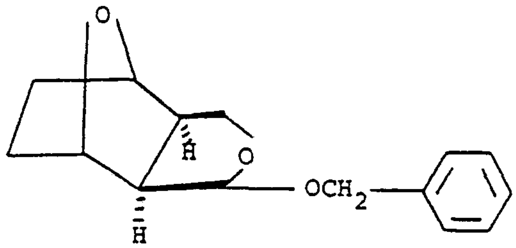

9. The compound as defined in Claim 1 having the formula

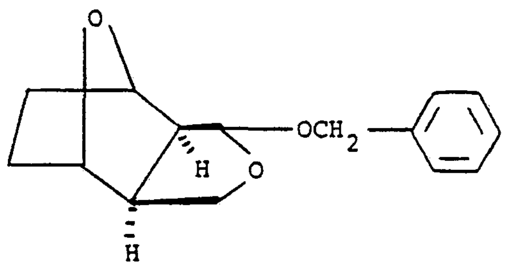

10. The compound as defined in Claim 1 having the formula

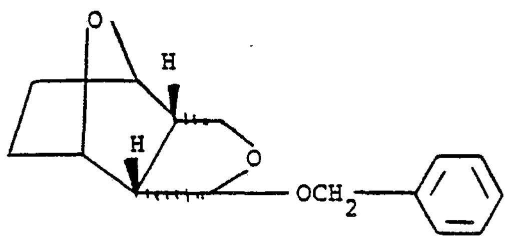

11. The compound as defined in Claim 1 having the formula

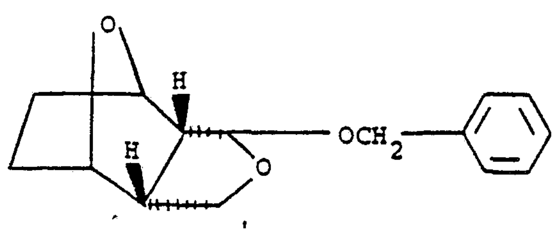

12. A method for preparing compounds as defined in Claim 1 which comprises esterifying a hemicacetal of the formula

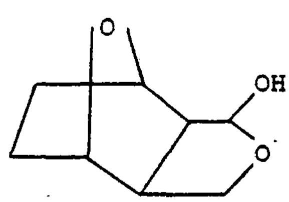

by reacting same with *d* or *l*-menthol to form a mixture of diastereomers, resolving the above mixture to recover optically active diastereomer of the structure

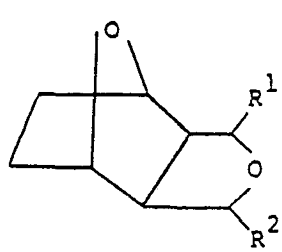

wherein one of $R^1$ and $R^2$ is 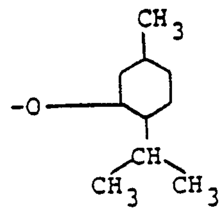

and the other is H, and reacting the above hemiacetal with benzyl alcohol to form the optically active compound

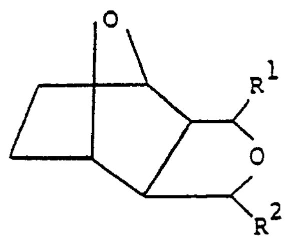

wherein one of $R^1$ and $R^2$ is 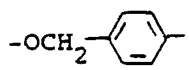 and the other is H.

13. The compound as defined in Claim 1 wherein $R^1$ is

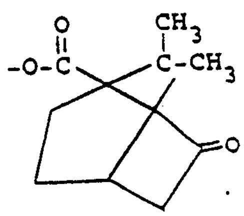

or

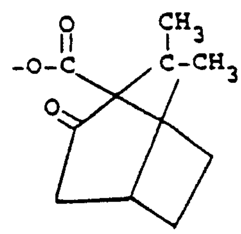

14. The compound as defined in Claim 1 wherein $R^2$ is

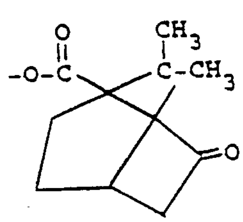

or

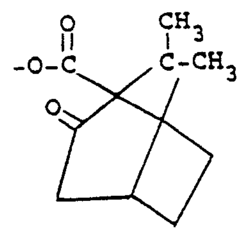

15. The compound as defined in Claim 1 having the structure

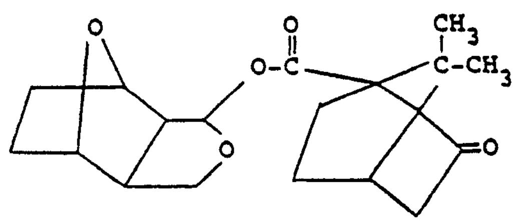

16. The compound as defined in Claim 1 having the structure

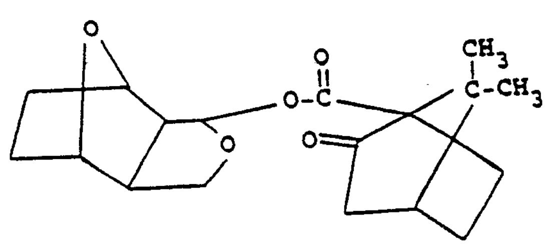

17. The compound as defined in Claim 1 having the structure

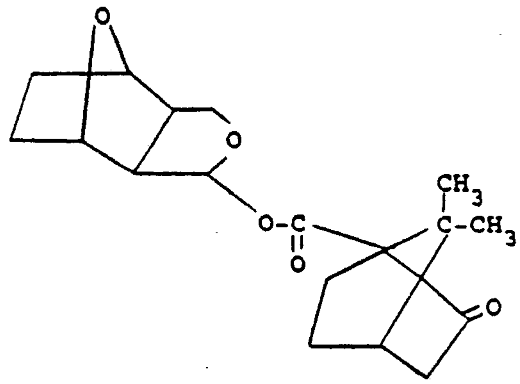

18. The compound as defined in Claim 1 having the structure

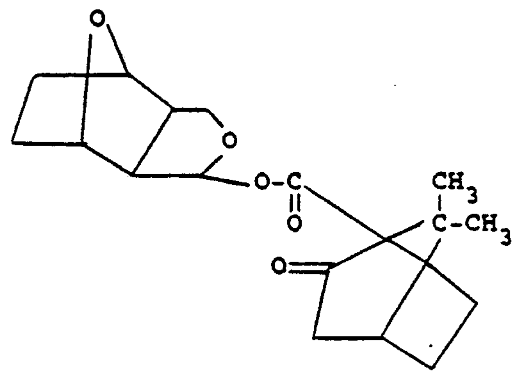

19. A method of preparing a ketopinate ester as defined in Claim 1 which comprises esterifying a hemiacetal of the formula

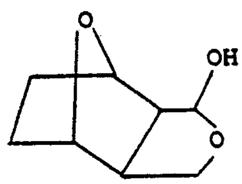

by reacting same with *d* or *l*-menthol to form a mixture of diastereomers, resolving the above mixture to recover 90% optically pure diastereomer of the structure

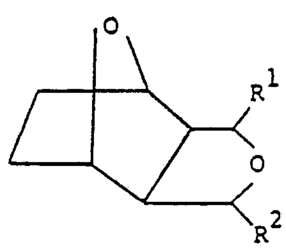 wherein $R^2$ is 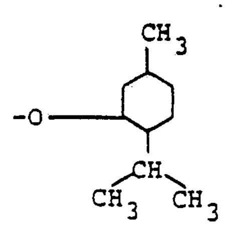

and $R^1$ is H, reacting the above hemiacetal with benzyl alcohol to form the optically active compound

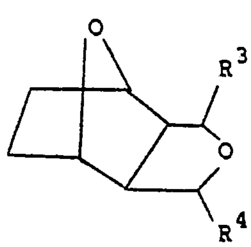 wherein $R^4$ is 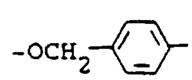

and $R^3$ is H, hydrogenating the benzyloxy compound to form a hemiacetal of the structure

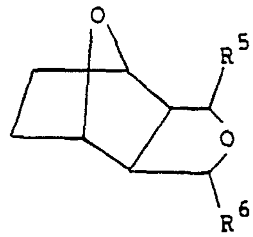

wherein $R^6$ is OH and $R^5$ is H, reacting the hemiacetal with *l* or *d*-ketopinic acid halide to form the optically active ketopinate ester.

20. The compound as defined in Claim 1 wherein $R^1$ is

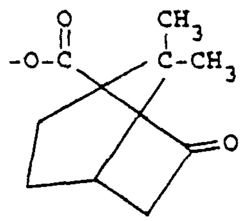

or

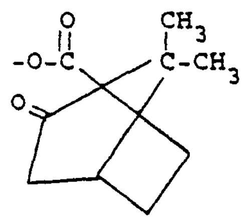

21. The compound as defined in Claim 1 wherein $R^2$ is

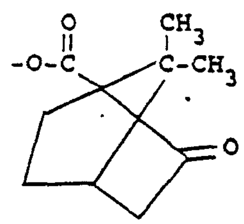

or

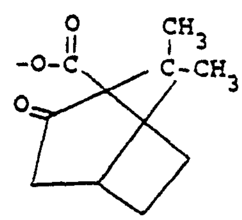

22. The compound as defined in Claim 1 having the structure

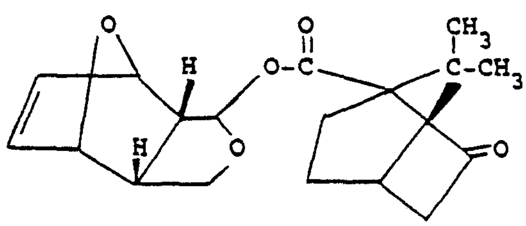

23. The compound as defined in Claim 1 having the structure

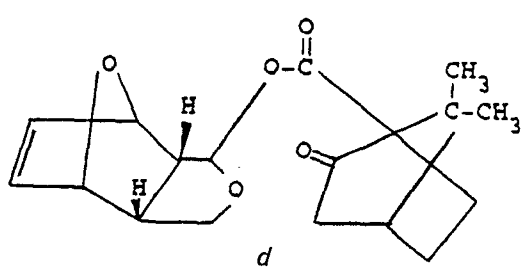

*d*

24. The compound as defined in Claim 1 having the structure

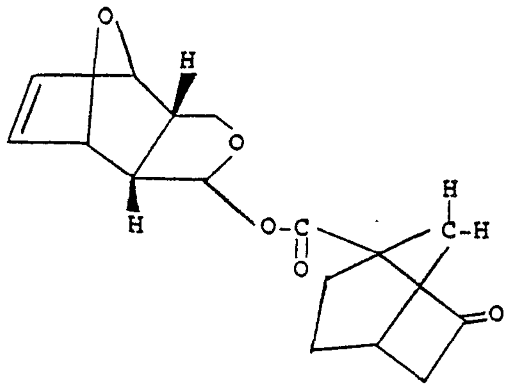

25. The compound as defined in Claim 1 having the structure

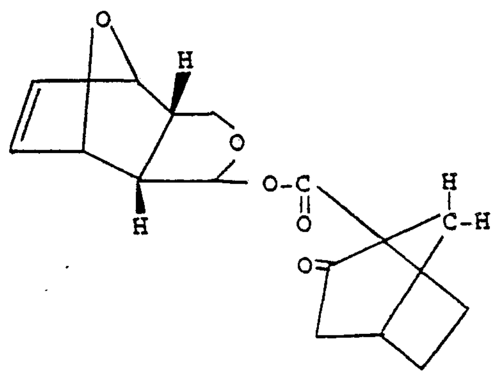

26. A method for preparing a compound as defined in Claim 1 which comprises reacting a racemic hemiacetal of the structure

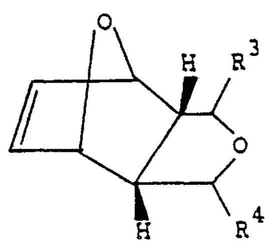

wherein one of $R^3$ and $R^4$ is OH and the other is H with *l* or *d*-ketopinic acid halide to form a racemic mixture of ketopinate esters and resolving the racemic ketopinate esters to recover the desired optically active ketopinate ester.

**Claims for the Contracting State: AT**

1. A method for preparing a ketopinate ester having the structure

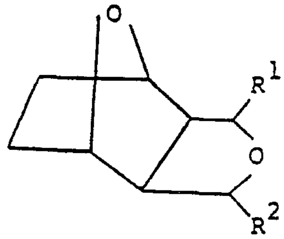

wherein one of $R^1$ and $R^2$ is 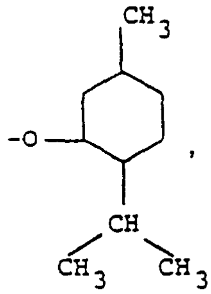 , 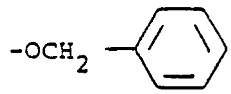 ,

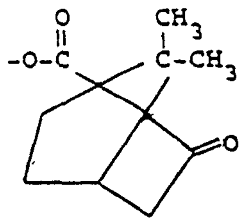 or 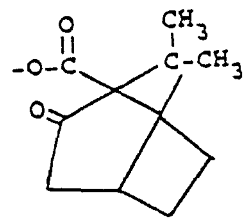

and the other is hydrogen, or having the structure 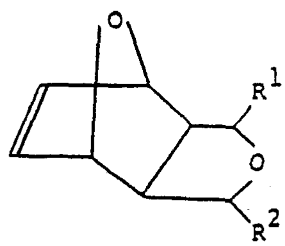

wherein one of $R^1$ and $R^2$ is 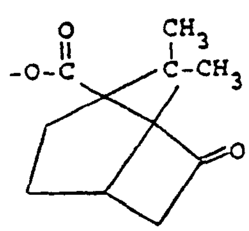 or 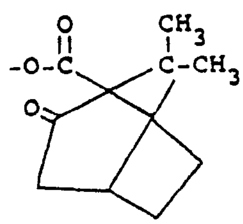

and the other is hydrogen which comprises esterifying a hemiacetal of the formula

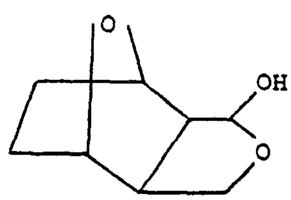

by reacting same with *d* or *l*-menthol to form a mixture of diastereomers, resolving the above mixture to recover 90% optically pure diastereomer of the structure

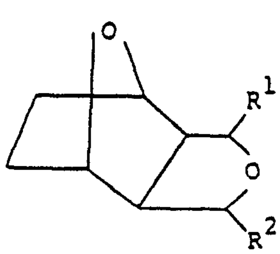 wherein $R^2$ is 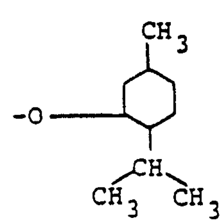

and $R^1$ is H, reacting the above hemiacetal with benzyl alcohol to form the optically active compound

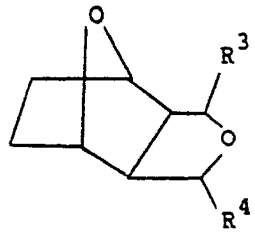 wherein $R^4$ is 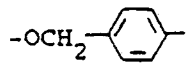

and $R^3$ is H, hydrogenating the benzyloxy compound to form a hemiacetal of the structure

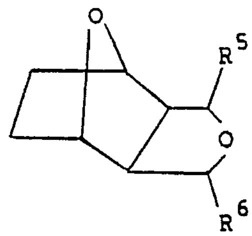

wherein $R^6$ is OH and $R^5$ is H, reacting the hemiacetal with *l* or *d*-ketopinic acid halide to form the optically active ketopinate ester.

2. A method for preparing a compound as defined in Claim 1 which comprises reacting a racemic hemiacetal of the structure

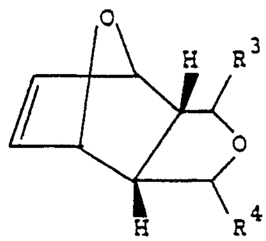

wherein one of $R^3$ and $R^4$ is OH and the other is H with *l* or *d*-ketopinic acid halide to form a racemic mixture of ketopinate esters and resolving the racemic ketopinate esters to recover the desired optically active ketopinate ester.

**Patentansprüche für die Vertragsstaaten: BE CH DE FR GB IT LI LU NL SE**

1. Eine Verbindung der Struktur 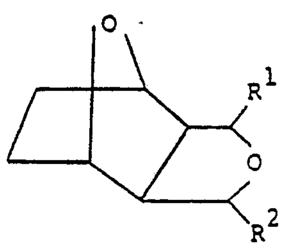

in der einer der Reste $R^1$ und $R^2$ eine der Gruppen

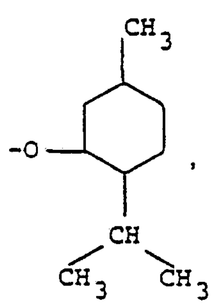 , 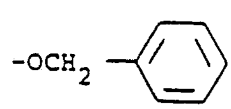 ,

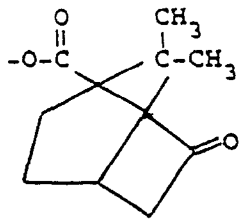

oder

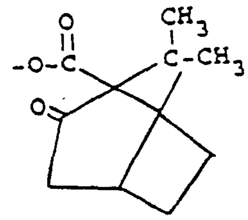

darstellt, und der andere Rest ein Wasserstoffatom ist, oder der Struktur

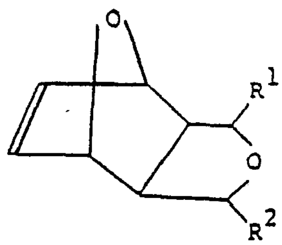

in der einer der Reste $R^1$ und $R^2$ eine der Gruppen

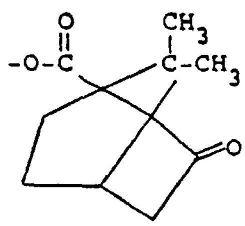

oder

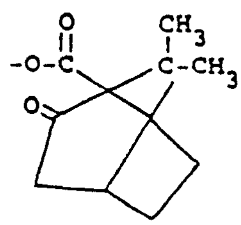

bedeutet, und der andere Rest ein Wasserstoffatom ist.

2. Verbindung nach Anspruch 1, in der $R^1$ ein Wasserstoffatom ist und $R^2$ eine der Gruppen

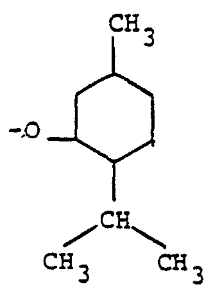

oder

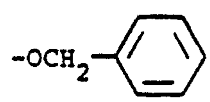

.

bedeutet.

3. Verbindung nach Anspruch 1, in der $R^2$ ein Wasserstoffatom ist und $R^1$ eine der Gruppen

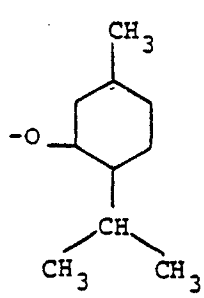

oder

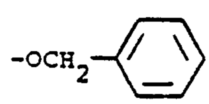

.

bedeutet.

4. Verbindung nach Anspruch 1 mit der Formel

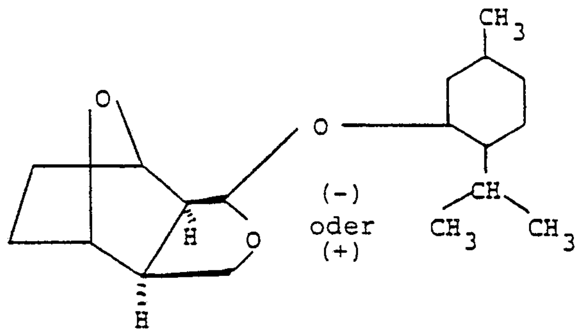

(-) oder (+)

5. Verbindung nach Anspruch 1 mit der Formel

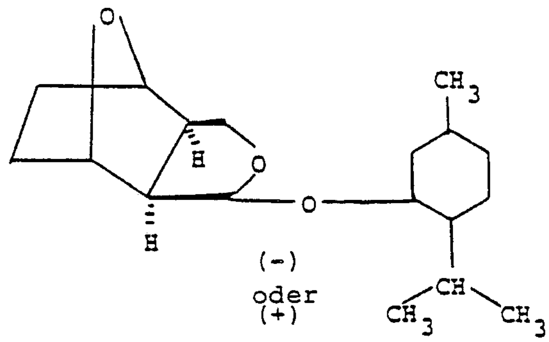

(-) oder (+)

6. Verbindung nach Anspruch 1 mit der Formel

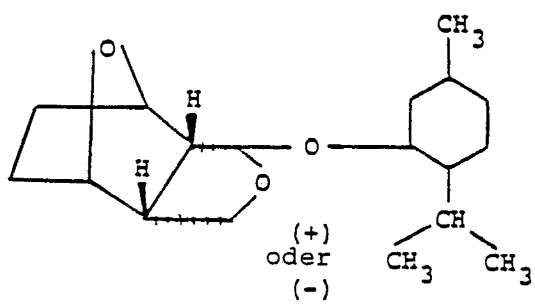

(+) oder (-)

7. Verbindung nach Anspruch 1 mit der Formel

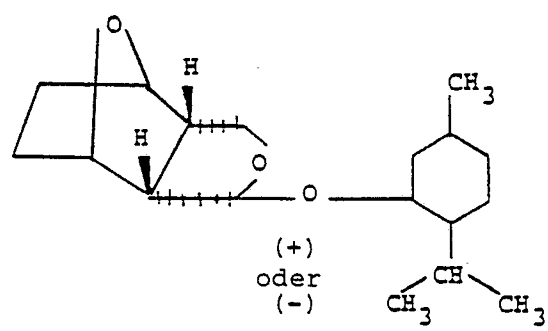

(+) oder (-)

8. Verbindung nach Anspruch 1 mit der Formel

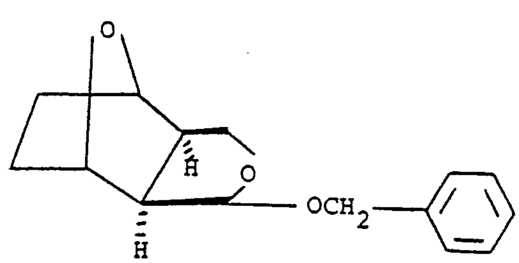

9. Verbindung nach Anspruch 1 mit der Formel

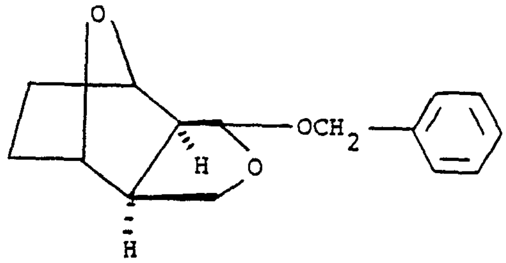

10. Verbindung nach Anspruch 1 mit der Formel

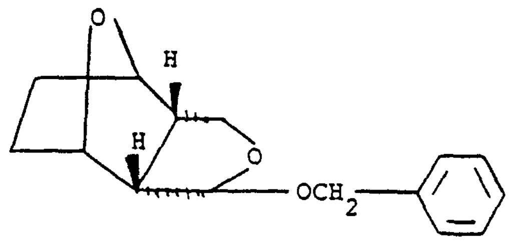

11. Verbindung nach Anspruch 1 mit der Formel

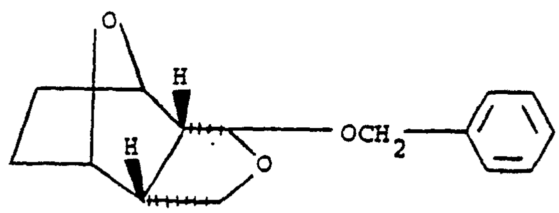

12. Verfahren zur Herstellung der Verbindungen nach Anspruch 1 durch Veresterung eines Hemiacetals der Formel

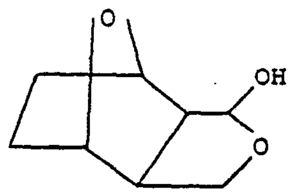

durch Umsetzung mit *d* oder *l*-Menthol zu einem Gemisch von Diastereomeren, Trennung des Gemisches zur Gewinnung des optisch aktiven Diastereomeren der Formel

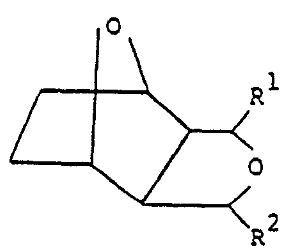

in der einer der Reste $R^1$ und $R^2$ die Gruppe 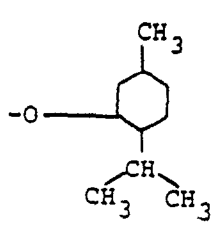

bedeutet und der andere Rest ein Wasserstoffatom darstellt, und Umsetzen des vorstehenden Hemiacetals mit Benzylalkohol zur Erzeugung der optisch aktiven Verbindung

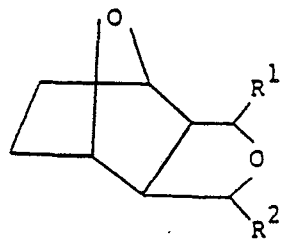

in der einer der Reste $R^1$ und $R^2$ die Gruppe 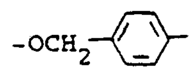 bedeutet und der andere Rest ein Wasserstoffatom darstellt.

13. Verbindung nach Anspruch 1, in der $R^1$ eine der Gruppen

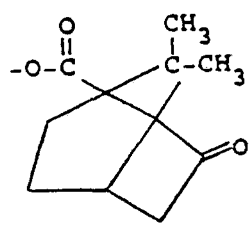 oder 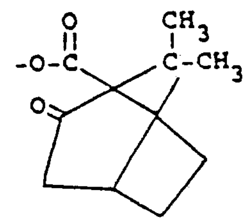

bedeutet.

14. Verbindung nach Anspruch 1, in der $R^2$ eine der Gruppen

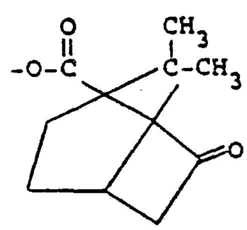 oder 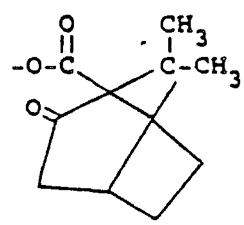

bedeutet.

15. Verbindung nach Anspruch 1 mit der Struktur

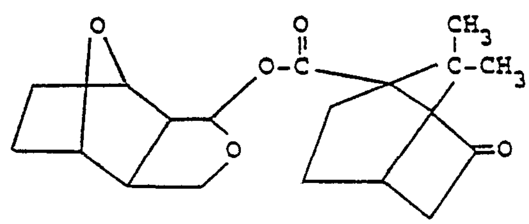

16. Verbindung nach Anspruch 1 mit der Struktur

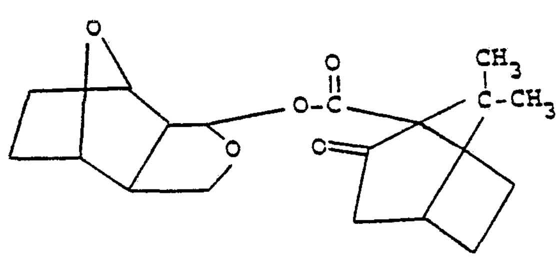

17. Verbindung nach Anspruch 1 mit der Struktur

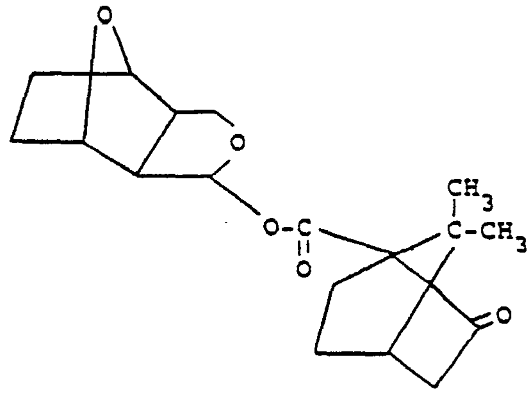

18. Verbindung nach Anspruch 1 mit der Struktur

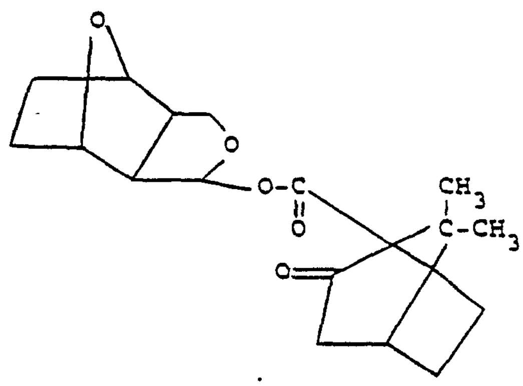

19. Verfahren zur Herstellung eines Ketopinat-Esters gemäß Anspruch 1 durch Veresterung eines Hemiacetals der Formel

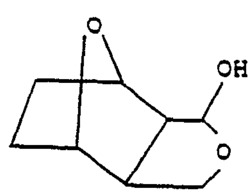

durch Behandlung mit *d* oder *l*-Menthol zur Erzeugung eines Diastereomerengemisches, Trennung des Gemisches zur Gewinnung von 90% optisch reinen Diastereomeren der Struktur

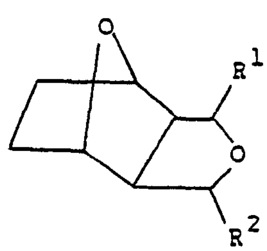

in der $R^2$ die Gruppe

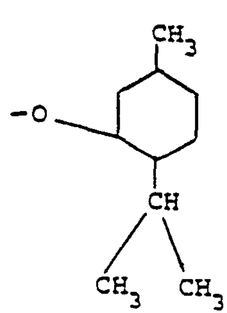

und $R^1$ ein Wasserstoffatom ist, Umsetzung des vorstehenden Hemiacetals mit Benzylalkohol zur Erzeugung der optisch aktiven Verbindung

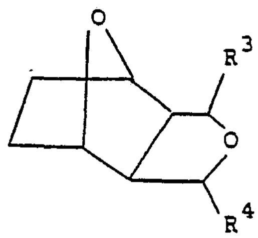

in der $R^4$ die Gruppe 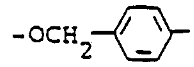

bedeutet und $R^3$ ein Wasserstoffatom darstellt, Hydrieren der Benzyloxyverbindung zur Erzeugung eines Hemiacetals der Struktur

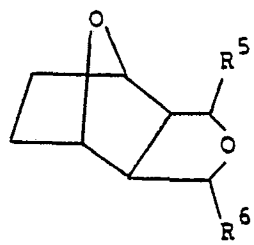

in der $R^6$ eine Hydroxylgruppe ist und $R^5$ ein Wasserstoffatom bedeutet und Umsetzung des Hemiacetals mit *l* oder *d*-Ketopinsäurehalogenid zur Herstellung des optisch aktiven Ketopinatesters.

20. Verbindung nach Anspruch 1, in der $R^1$ eine der Gruppen

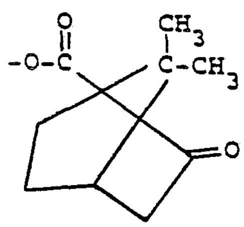 oder 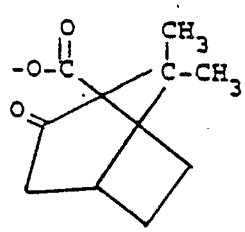

bedeutet.

21. Verbindung nach Anspruch 1, in der $R^2$ eine der Gruppen

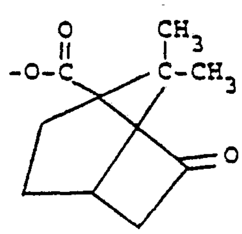 oder 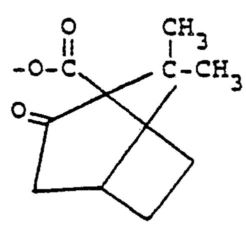

bedeutet.

22. Verbindung nach Anspruch 1 der Struktur

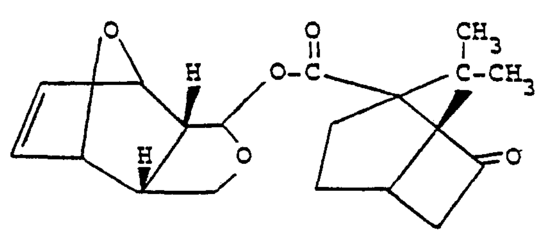

23. Verbindung nach Anspruch 1 der Struktur

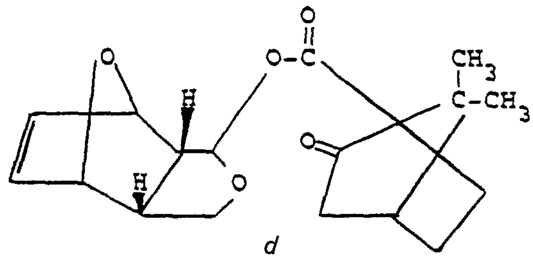

*d*

24. Verbindung nach Anspruch 1 der Struktur

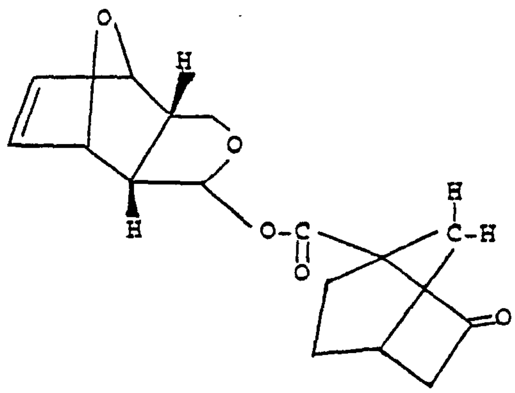

25. Verbindung nach Anspruch 1 der Struktur

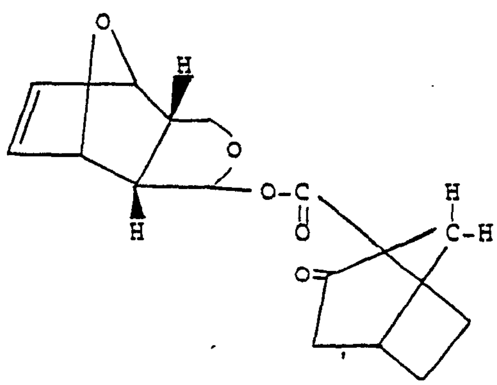

26. Verfahren zur Herstellung einer Verbindung nach Anspruch 1 durch Umsetzung eines racemischen Hemiacetals der Struktur

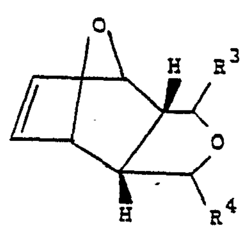

in der einer der Reste $R^3$ und $R^4$ eine Hydroxylgruppe bedeutet und der andere ein Wasserstoffatom darstellt, mit *l*- oder *d*-Ketopinsäurehalogenid zur Erzeugung eines racemischen Gemisches von Ketopinatestern und Trennung der racemischen Ketopinatester zur Gewinnung des gewünschten optisch aktiven Ketopinatesters.

**Patentansprüche für den Vertragsstaat: AT**

1. Verfahren zur Herstellung eines Ketopinatesters mit der Struktur

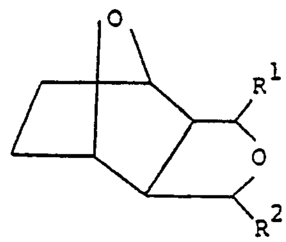

in der einer der Reste $R^1$ und $R^2$ eine der Gruppen 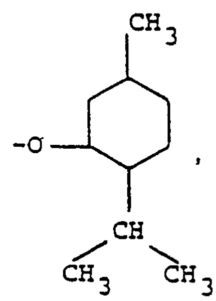 , 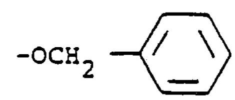 ,

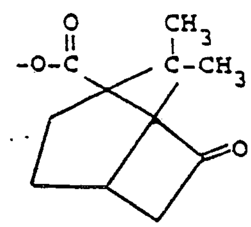 oder 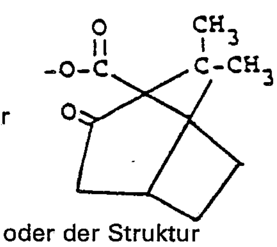

darstellt, und der andere Rest ein Wasserstoffatom ist, oder der Struktur

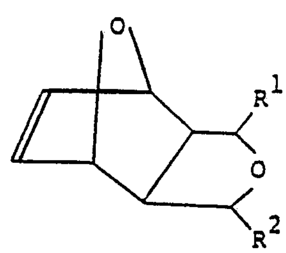

in der einer der Reste $R^1$ und $R^2$ eine der Gruppen

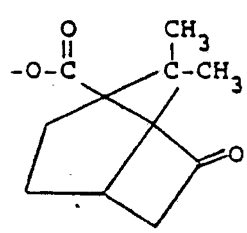 oder 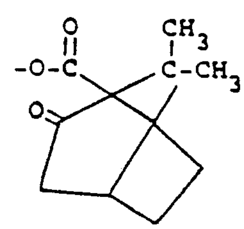

bedeutet, und der andere Rest ein Wasserstoffatom ist, durch Veresterung eines Hemiacetals der Formel

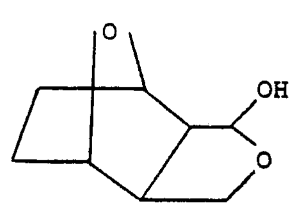

durch Behandlung mit *d* oder *l*-Menthol zur Erzeugung eines Diastereomerengemisches, Trennung des Gemisches zur Gewinnung von 90% optisch reinen Diastereomeren der Struktur

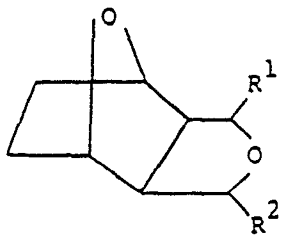 in der $R^2$ die Gruppe 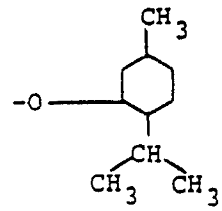

und $R^1$ ein Wasserstoffatom ist, Umsetzung des vorstehenden Hemiacetals mit Benzylalkohol zur Erzeugung der optisch aktiven Verbindung

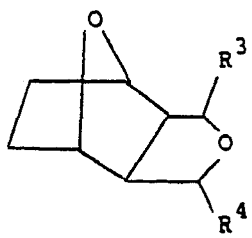 in der $R^4$ die Gruppe 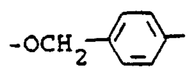

bedeutet und $R^3$ ein Wasserstoffatom darstellt, Hydrieren der Benzyloxyverbindung zur Erzeugung eines Hemiacetals der Struktur

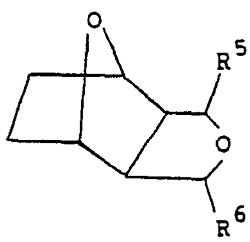

in der $R^6$ eine Hydroxylgruppe ist und $R^5$ ein Wasserstoffatom bedeutet und Umsetzung des Hemiacetals mit *l* oder *d*-Ketopinsäurehalogenid zur Herstellung des optisch aktiven Ketopinatesters.

2. Verfahren zur Herstellung einer Verbindung nach Anspruch 1 durch Umsetzung eines racemischen Hemiacetals der Struktur

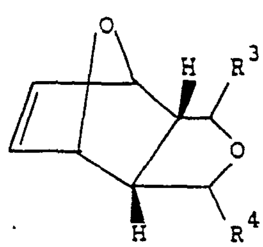

in der einer der Reste $R^3$ und $R^4$ eine Hydroxylgruppe bedeutet und der andere ein Wasserstoffatom darstellt, mit *l*- oder *d*-Ketopinsäurehalogenid zur Erzeugung eines racemischen Gemisches von Ketopinatestern und Trennung der racemischen Ketopinatester zur Gewinnung des gewünschten optisch aktiven Ketopinatesters.

**Revendications pour les Etats contractants: BE CH DE FR GB IT LI LU NL SE**

1. Composé de formule

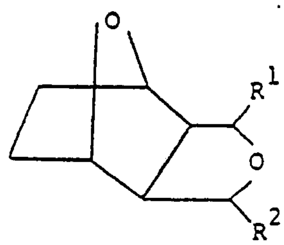

dans laquelle l'un de $R^1$ et $R^2$ est

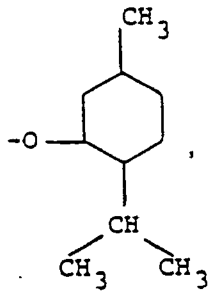

,

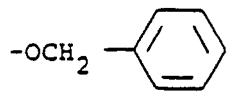

,

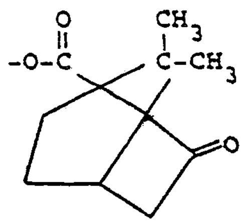

ou

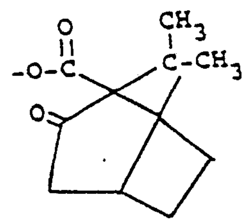

et l'autre est l'hydrogène, ou bien de formule

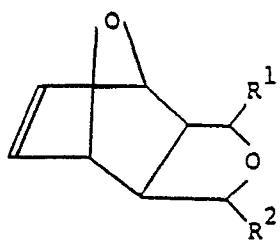

dans laquelle l'un de $R^1$ et $R^2$ est

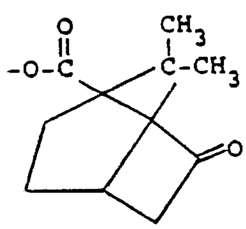

ou

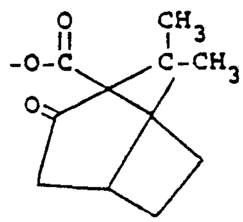

et l'autre est l'hydrogène.

2. Composé selon la revendication 1, dans la formule duquel $R^1$ est l'hydrogène et $R^2$ est

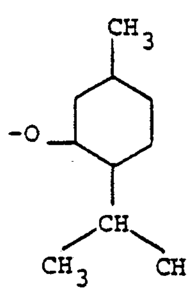

ou

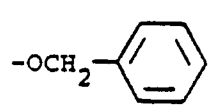

.

3. Composé selon la revendication 1, dans laquelle $R^2$ est l'hydrogène et $R^1$ est

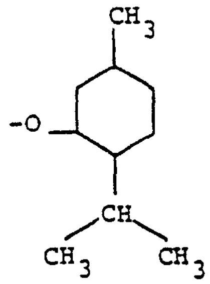

ou

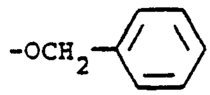

.

4. Composé selon la revendication 1, ayant pour formule

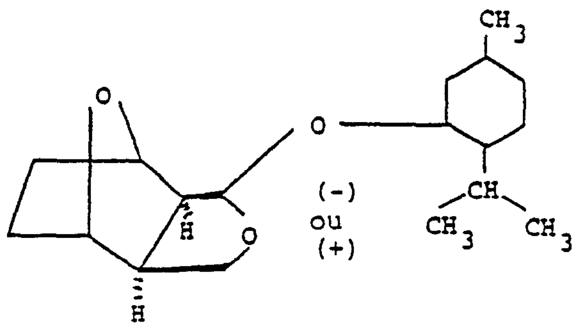

(-)
ou
(+)

.

5. Composé selon la revendication 1, ayant pour formule

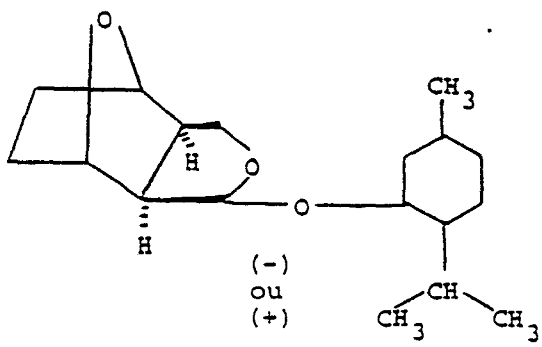

(-)
ou
(+)

6. Composé selon la revendication 1, ayant pour formule

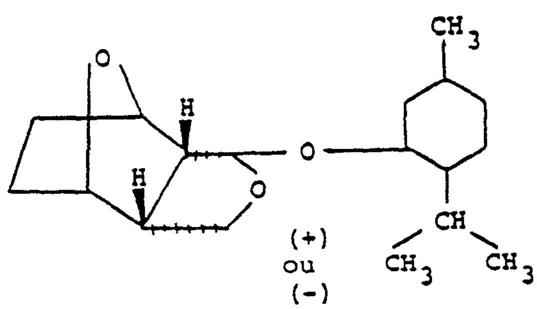

(+)
ou
(-)

.

7. Composé selon la revendication 1, ayant pour formule

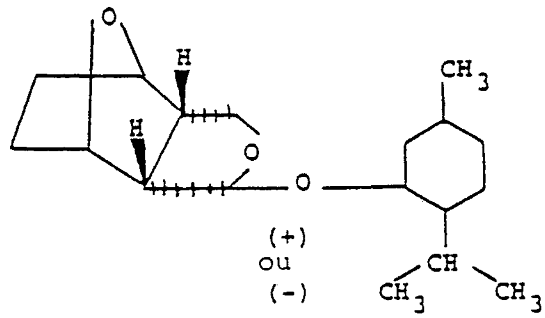

(+) ou (-)

8. Composé selon la revendication 1, ayant pour formule

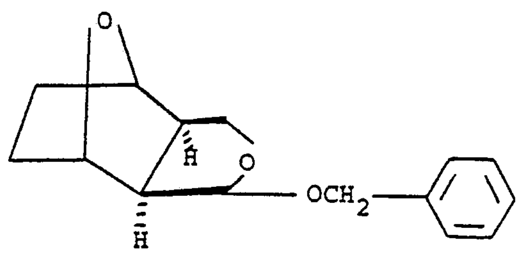

9. Composé selon la revendication 1, ayant pour formule

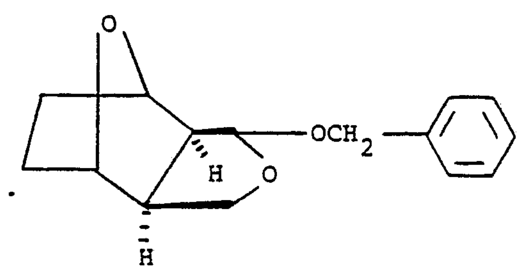

10. Composé selon la revendication 1, ayant pour formule

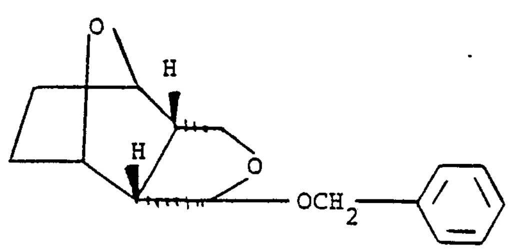

11. Composé selon la revendication 1, ayant pour formule

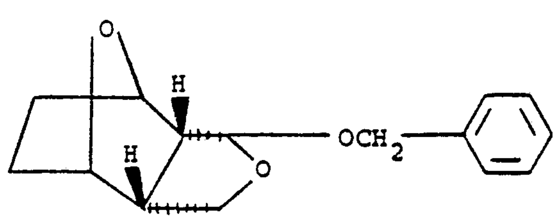

12. Procédé de préparation de composés tels qu'ils sont définis dans la revendication 1, qui consiste à estérifier un hémiacétal de formule

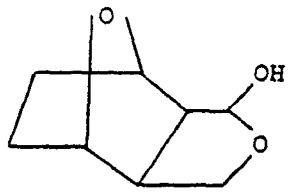

en le faisant réagir avec du *d* ou du *l*-menthol pour former un mélange de diastéréo-isomères, à dédoubler le mélange précédent pour récupérer le diastéréo-isomère optiquement actif, de formule

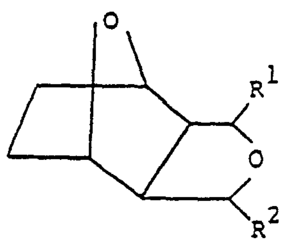 dans laquelle l'un de $R^1$ et $R^2$ est 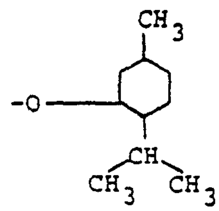

et l'autre est l'hydrogène, et à faire réagir l'hémiacétal précédent avec de l'alcool benzylique pour former le composé optiquement actif de formule

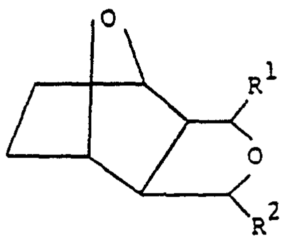 dans laquelle l'un de $R^1$ et $R^2$ est 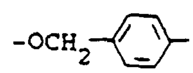

et l'autre est l'hydrogène.

13. Composé selon la revendication 1, dans la formule duquel $R^1$ est

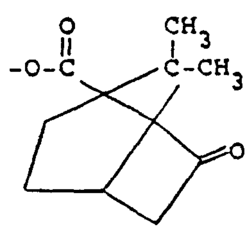 ou 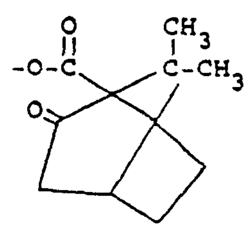

14. Composé selon la revendication 1, dans la formule duquel $R^2$ est

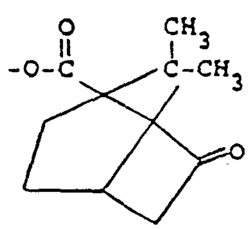 ou 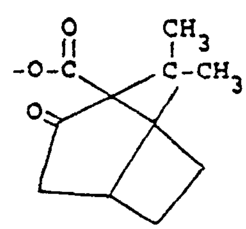

15. Composé selon la revendication 1, ayant pour formule

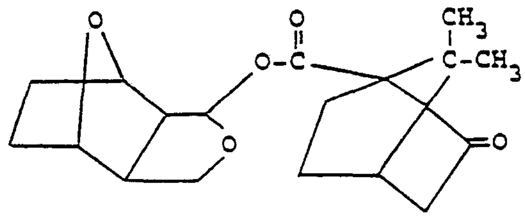

16. Composé selon la revendication 1, ayant pour formule

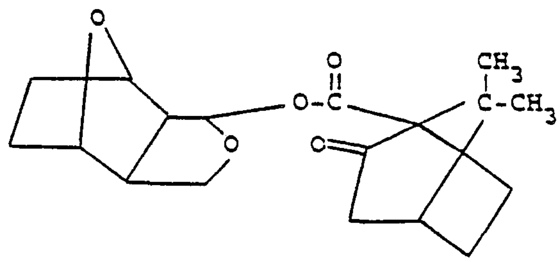

17. Composé selon la revendication 1, ayant pour formule

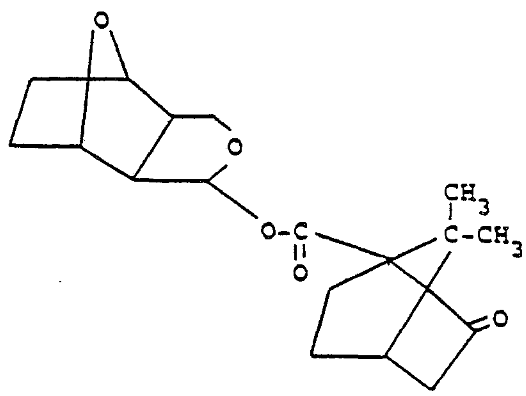

18. Composé selon la revendication 1, ayant pour formule

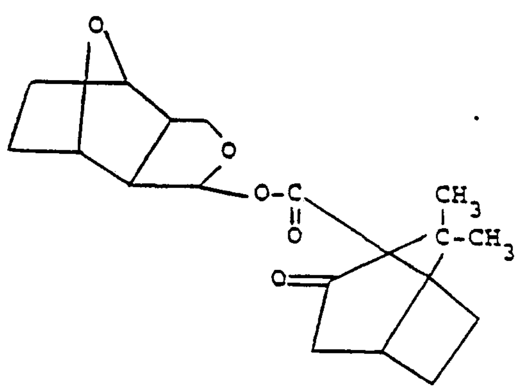

19. Procédé de préparation d'un cétopinate tel qu'il est défini dans la revendication 1, qui consiste à estérifier un hémiacétal de formule

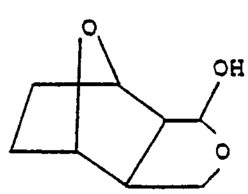

en le faisant réagir avec du *d*- ou *l*-menthol pour former un mélange de diastéréo-isomères, à dédoubler le mélange précédent pour récupérer un diastéréo-isomère optiquement pur à 90%, de formule

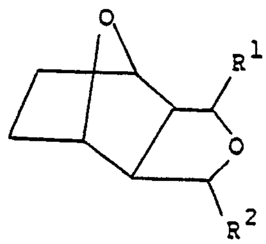

dans laquelle $R^2$ est

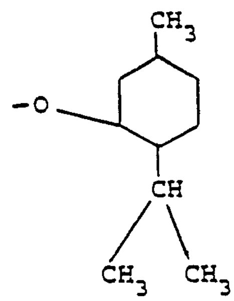

et $R^1$ est l'hydrogène, à faire réagir l'hémiacétal précédent avec de l'alcool benzylique pour former le composé optiquement actif de formule

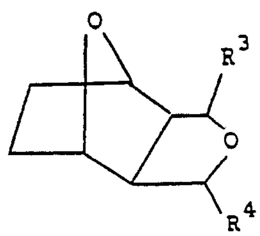

dans laquelle $R^4$ est

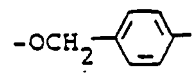

et $R^3$ est l'hydrogène, à hydrogéner le composé benzyloxy pour former un hémiacétal de formule

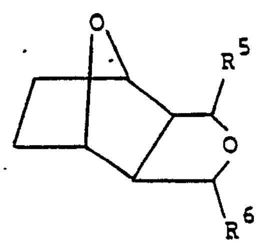

dans laquelle $R^6$ est OH et $R^5$ est l'hydrogène, et à faire réagir l'hémiacétal avec un halogénure d'acide *l*- ou *d*-cétopinique pour former le cétopinate optiquement actif.

20. Composé selon la revendication 1, dans la formule duquel $R^1$ est

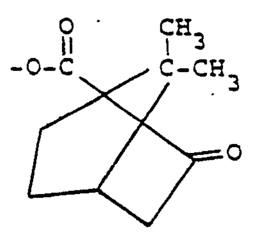

ou

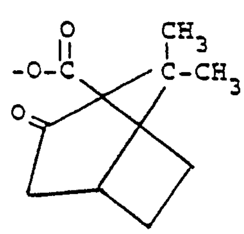

21. Composé selon la revendication 1, dans la formule duquel $R^2$ est

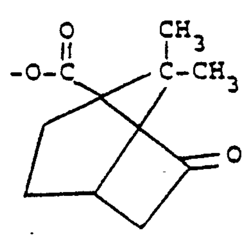

ou

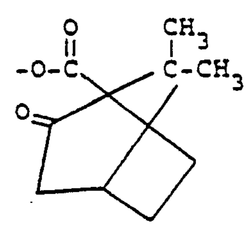

22. Composé selon la revendication 1, ayant pour formule

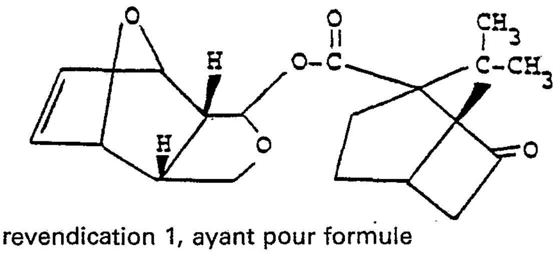

23. Composé selon la revendication 1, ayant pour formule

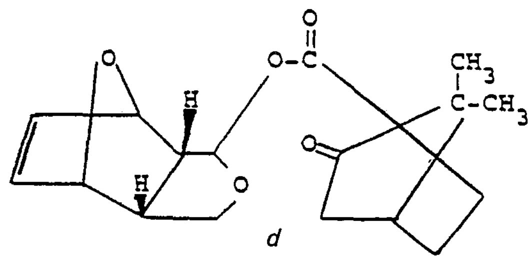

24. Composé selon la revendication 1, ayant pour formule

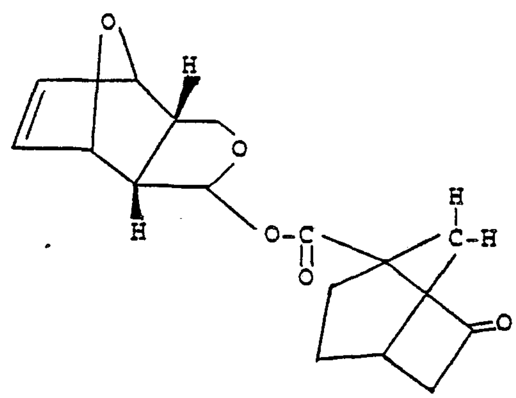

25. Composé selon la revendication 1, ayant pour formule

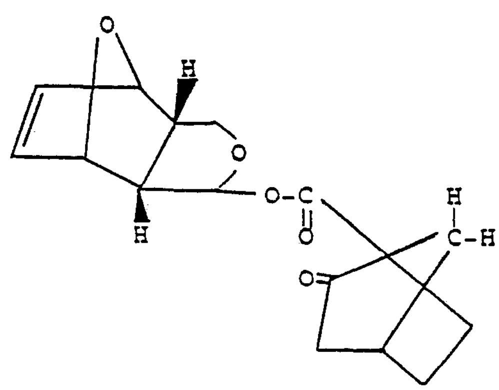

26. Procédé de préparation d'un composé tel qu'il est défini dans la revendication 1, qui consiste à faire réagir un hémiacétal racémique de formule

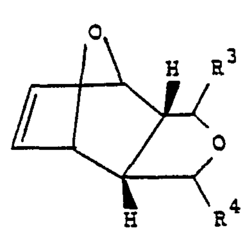

dans laquelle l'un de $R^3$ et $R^4$ est OH et l'autre est l'hydrogène, avec un halogénure d'acide *l*- ou *d*-cétopinique pour former un mélange racémique de cétopinates, et à dédoubler les cétopinates racémiques pour récupérer le cétopinate optiquement actif voulu.

**Revendications pour l'Etat contractant: AT**

1. Procédé de préparation d'un cétopinate ayant pour formule

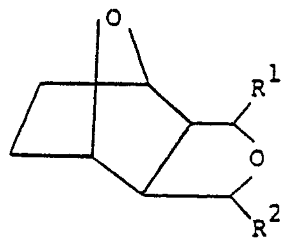

dans laquelle l'un de $R^1$ et $R^2$ est 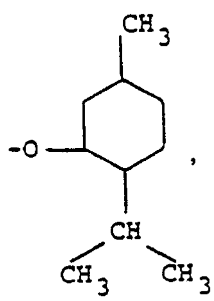 , 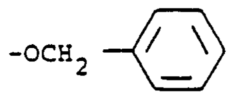 ,

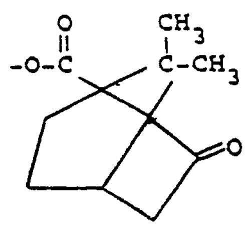 ou 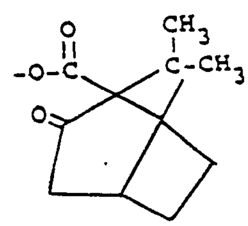

et l'autre est l'hydrogène, ou bien ayant pour formule 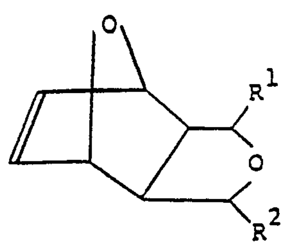

dans laquelle l'un de $R^1$ et $R^2$ est 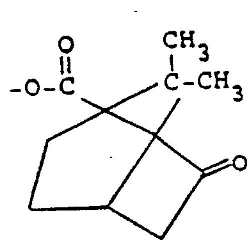 ou 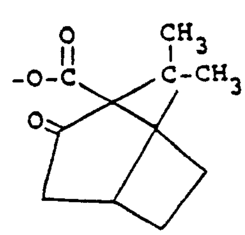

et l'autre est l'hydrogène,

qui consiste à estérifier un hémiacétal de formule

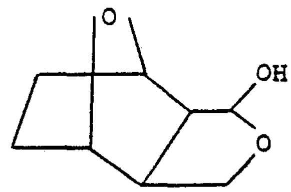

en le faisant réagir avec du *d*- ou *l*-menthol pour former un mélange de diastéréo-isomères, à dédoubler le mélange précédent pour récupérer un diastéréo-isomère optiquement pur à 90%, de formule

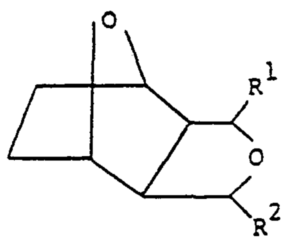 dans laquelle $R^2$ est 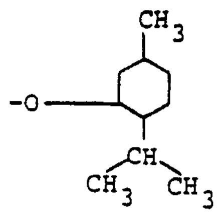

et $R^1$ est l'hydrogène, à faire réagir l'hémiacétal précédent avec de l'alcool benzylique pour former le composé optiquement actif de formule

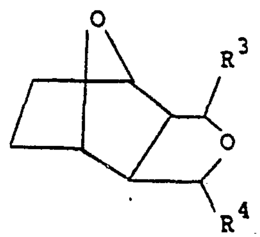 dans laquelle $R^4$ est 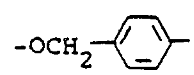

et $R^3$ est l'hydrogène, à hydrogéner le composé benzyloxy pour former un hémiacétal de formule

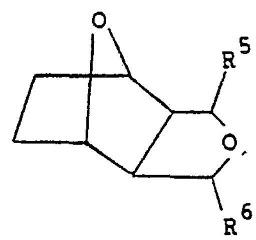

dans laquelle $R^6$ est OH et $R^5$ est l'hydrogène, et à faire réagir l'hémiacétal avec un halogénure d'acide *l*- ou *d*-cétopinique pour former le cétopinate optiquement actif.

2. Procédé de préparation d'un composé tel qu'il est défini dans la revendication 1, qui consiste à faire réagir un hémiacétal racémique de formule

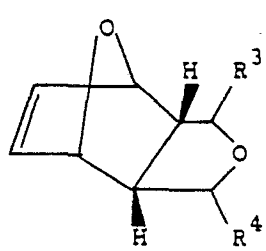

dans laquelle l'un de $R^3$ et $R^4$ est OH et l'autre est l'hydrogène, avec un halogénure d'acide *l*- ou *d*-cétopinique pour former un mélange racémique de cétopinates, et à dédouble les cétopinates racémiques pour récupérer le cétopinate optiquement actif voulu.